# EUROPEAN PATENT APPLICATION

(11) **EP 4 252 750 A1**
(43) Date of publication of application: **04.10.2023**
(21) Application number: 22166017.8
(22) Date of filing: 31.03.2022
(51) Int. Cl.: A61K 31/285, A61K 31/29, A61K 31/137, A61K 9/00, A61K 31/145, A61K 31/197, A61P 25/28

(54) **PHARMACEUTICAL PRESERVATION OF CREB ACTIVATION IN THE TREATMENT OF ALZHEIMER'S DISEASE**

(71) Applicant: Leibniz-Institut für Neurobiologie, 39118 Magdeburg (DE); Westfälische Wilhelms-Universität Münster, 48149 Münster (DE)
(72) Inventor: Kreutz, Michael, 39112 Magdeburg (DE); Reissner, Carsten, 48161 Münster (DE); Grochowska, Katarzyna, 22085 Hamburg (DE); Oelschlegel, Anja, 39122 Magdeburg (DE); Karpova, Anna, 39104 Magdeburg (DE); Gomes, Guilherme, 39116 Magdeburg (DE)
(74) Representative: Hertin und Partner Rechts- und Patentanwälte PartG mbB

(57) **Abstract**

The invention relates to a compound nitarsone, or derivative or salt thereof for use in the treatment of a neurodegenerative disease, such as Alzheimer's disease (AD), dementia, Parkinson's disease (PD) or amyotrophic lateral sclerosis (ALS). The invention further relates to a pharmaceutical composition comprising the compound, or derivative or salt thereof for use according to the invention.

## Description

The invention relates to a compound nitarsone, or derivative or salt thereof for use in the treatment of a neurodegenerative disease, such as Alzheimer's disease (AD), dementia, Parkinson's disease (PD) or amyotrophic lateral sclerosis (ALS). The invention further relates to a pharmaceutical composition comprising the compound nitarsone, or derivative or salt thereof according to the invention.

### BACKGROUND OF THE INVENTION

Soluble oligomeric Aβ induces deterioration of synaptic function in Alzheimer's disease (AD) even before overt signs of dementia and plaque formation (Forner et al., 2017; Li and Selkoe, 2020; Selkoe, 2002; Selkoe and Hardy, 2016). While a large number of Aβ receptors have been suggested (Jarosz-Griffiths et al., 2016) their pathophysiological relevance for synaptic dysfunction in vivo is still elusive given that the earliest hallmark of AD in humans and animal models is neuronal hyperexcitability caused by suppression of glutamate reuptake (Zott et al., 2019). In this scenario glutamate spillover to perisynaptic sites might cause detrimental activation of extrasynaptic N-Methyl-D-Aspartate receptors (NMDAR).

NMDAR are heteromeric glutamate-gated ion channels implicated in synaptic plasticity, learning and memory but also in neurodegeneration and excitotoxicity (Bading, 2017; Hardingham and Bading, 2010; Parsons and Raymond, 2014). A prevailing hypothesis suggests that the opposing functions of NMDAR can be attributed to their subunit composition and subcellular localization (Bading, 2017). Aberrant and synergistic activation of GluN2B-containing NMDAR at extrasynaptic sites by glutamate and Aβ-amyloidosis has been implicated in AD (Bading, 2017; Bordji et al., 2010; Malinow, 2012; Marcello et al., 2018). Signaling downstream of synaptic and extrasynaptic NMDAR is tightly and antagonistically coupled to the transcription factor CREB. Activation of synaptic NMDAR activates CREB through sustained phosphorylation of a crucial serine at position 133 (S133) and thereby promotes the expression of plasticity-related genes (Hardingham and Bading, 2010) critically involved in learning and memory (Barco et al., 2002; Carlezon et al., 2005). Conversely, predominant activation of extrasynaptic NMDAR leads to sustained dephosphorylation of CREB (CREB shutoff), rendering CREB transcriptionally inactive (Hardingham et al., 2002). Loss of CREB-dependent gene expression after extrasynaptic NMDAR activation precedes cell death and neurodegeneration (Hardingham and Bading, 2010) and Aβ-induced CREB shutoff plays a role already in early synaptic dysfunction driving cognitive impairment in AD (Bartolotti et al., 2016; Espana et al., 2010; Saura and Valero, 2011; Teich et al., 2015; Yiu et al., 2011). Under the premise that activation of extrasynaptic NMDAR happens before the manifestation of clinical symptoms it is very likely that Aβ will interfere already at this stage with transcriptional regulation. Surprisingly little is known, however, on mechanisms of CREB shutoff in general and in AD in particular.

In previous work, the inventors found that the synapto-nuclear messenger Jacob, following long-distance transport and nuclear import, transduces the synaptic and extrasynaptic origin of NMDAR signals to the nucleus (Dieterich et al., 2008; Grochowska et al., 2021; Karpova et al., 2013; Panayotis et al., 2015). Activation of synaptic NMDARs leads to phosphorylation of Jacob at serine 180 (S180) via MAP-kinase ERK1/2, which is followed by trafficking of a pJacob/pERK1/2 signalosome along microtubules to neuronal nuclei (Karpova et al., 2013). Binding of the intermediate filament α-internexin protects pJacob and pERK against dephosphorylation during transport (Karpova et al., 2013).

This signalosome promotes CREB phosphorylation at S133 and hence CREB-dependent gene expression (Karpova et al., 2013). On the contrary, activation of extrasynaptic NMDAR leads to prominent translocation of non-phosphorylated Jacob and induces CREB shutoff followed by stripping of synaptic contacts, simplification of dendritic arborization, and ultimately cell death (Gomes et al., 2014; Grochowska et al., 2017; Rönicke et al., 2011). Collectively, these data suggest that Jacob operates as a mobile signalling hub that docks NMDAR-derived signalosomes to nuclear target sites (Dieterich et al., 2008; Grochowska et al., 2021; Karpova et al., 2013; Marcello et al., 2018; Panayotis et al., 2015).

In summary, the onset of certain neurodegenerative diseases, such as Alzheimer's disease, is characterized by impairment of synaptic function that is caused by amyloid-β oligomers-induced synaptotoxicity. Synaptotoxicity is likely also a consequence of the disruption in the expression of plasticity-related genes.

Despite the years of intensive studies aimed at understanding of the processes contributing to AD, no successful, synapto- and neuro-protective therapies for AD or other neurodegenerative diseases have been found yet.

Soluble amyloid-β oligomers are the key agents inducing disturbance of neuronal gene expression governed by the transcription factor CREB. CREB is fundamentally involved in plasticity-related gene expression and its inactivation leads to severe impairment in synaptic function and, ultimately, neurodegeneration in AD or other neurodegenerative diseases. No treatments are available yet to prevent amyloid-β-induced transcriptional inactivation of CREB in AD.

### SUMMARY OF THE INVENTION

In light of the prior art the technical problem underlying the present invention is to provide means for the treatment, prevention, inhibition or amelioration of symptoms of neurodegenerative diseases, such as Alzheimer's disease (AD) and related conditions, wherein the transcriptional inactivation of CREB plays a role in disease progression.

This problem is solved by the features of the independent claims. Preferred embodiments of the present invention are provided by the dependent claims.

The present invention provides improved means for inhibiting Jacob-induced inactivation of the transcription factor CREB through a small chemical compound nitarsone, or derivatives or structural analogues or salts thereof for use in the treatment, prevention, inhibition of neurodegenerative diseases such as Alzheimer's disease (AD) and related conditions and/or the amelioration of symptoms thereof, wherein preferably the transcriptional inactivation of CREB plays a role in disease progression.

The invention relates to a compound nitarsone, or derivative or salt thereof for use in the treatment of a neurodegenerative disease in a subject.

In embodiments the invention relates to a compound nitarsone, or derivative or salt thereof for use in the treatment of a confirmed or suspected neurodegenerative disease in a subject.

In embodiments the invention relates to a compound nitarsone, or derivative or salt thereof for use in the treatment of a neurodegenerative disease in a subject to prevent and/or ameliorate the symptoms of said neurodegenerative disease.

In embodiments the invention relates to the compound nitarsone, or derivative or salt thereof for use in the treatment of a neurodegenerative disease in a subject, wherein the subject is suspected of having or has been diagnosed with a neurodegenerative disease.

The invention also relates to a compound nitarsone, or derivative or salt thereof for use in the treatment of a neurological disease in a subject.

In embodiments of the invention the neurological disease is Alzheimer's Disease (AD), dementia, Parkinson's disease (PD) or amyotrophic lateral sclerosis (ALS).

The invention further relates to a compound nitarsone, or derivative or salt thereof for use as a medicament.

The invention also relates to a pharmaceutical composition comprising a compound nitarsone, or derivative or salt thereof for use in the treatment of a neurodegenerative disease in a subject.

The invention further relates to a pharmaceutical composition comprising a compound nitarsone, or derivative or salt thereof for use in the treatment of a confirmed or suspected neurodegenerative disease in a subject.

In preferred embodiments of the invention the neurodegenerative disease is Alzheimer's Disease (AD), dementia, Parkinson's disease (PD) or amyotrophic lateral sclerosis (ALS).

Synaptic dysfunction caused by soluble β-Amytoid (Aβ) is a hallmark of the early stage of Alzheimer's disease (AD) and is tightly linked to cognitive decline. Unfortunately, today only few pharmaceutical approaches are available to target the molecular dysfunctions underlying neurodegenerative diseases, such as AD or dementia. The inventors surprisingly found, that nitarsone is able to prevent impairment of synaptic plasticity as well as cognitive decline associated with neurodegenerative diseases such as AD and dementia.

The examples herein demonstrate the unexpected capability of nitarsone and pharmaceutical compositions comprising nitarsone to prevent impairment of synaptic plasticity as well as cognitive decline in models of AD. The inventors found that one of the mechanisms of how the small chemical compound nitarsone can cause its unexpected beneficial effects in the treatment of neurodegenerative diseases is that it selectively hinders the molecular signaling mechanisms underlying neuronal cell survival and plasticity and that treatment with nitarsone preserves and restores the transcriptional activity of the master regulator protein CREB (cAMP-responsive element-binding protein).

Accordingly, embodiments of the present invention provide means and methods for treating, preventing, disrupting and/or reverting inhibition in CREB activity linked to a neurodegenerative condition. Such CREB inhibition can lead to synaptic stripping as well as neuronal loss underlying memory impairment typical for patients suffering from neurodegenerative diseases, such as AD and dementia.

The inventors have previously described a macromolecular complex containing the protein Jacob which translocates from synaptic sites to the nucleus and directly induces CREB inactivation. Jacob is a synapto-nuclear protein messenger, which plays an important role in linking synaptic activity to nuclear gene expression in plasticity-related signaling as well as neurodegeneration. Jacob is primarily found in the neuronal nucleus and synapses of excitatory neurons in the forebrain, where it is involved in signaling pathways associated with N-methyl-D-aspartic acid receptors (NMDARs). Jacob drives the macromolecular complexes consisting of a kinase or a phosphatase capable of activating or inactivating CREB from NMDAR activation sites and docks them to CREB in the nucleus. Followed by amyloid-β activation of NMDAR Jacob displaces LMO4, a transcriptional co-activator of CREB, rendering it transcriptionally inactive and susceptible for degradation. Importantly, this type of displacement is characteristic for prolonged neurodegenerative signaling.

The detailed knowledge about this signaling cascade obtained by the inventors enables precise targeting of the pathological process, preventing displacement of LMO4 from CREB and its inactivation. In consequence, the inventors performed molecular modeling of the binding interfaces between LMO4, CREB, and Jacob. Based on their knowledge the inventors hypothesized that blocking the displacement of LMO4 from CREB by non-phosphorylated Jacob should not interfere with the interaction with LMO4 with CREB. The inventors predicted that this molecular mechanism should preserve the transcriptional activity of CREB despite the presence of non-phosphorylated Jacob in the CREB complex.

Subsequently, they screened small chemical compound libraries to identify substances that might be capable to prevent Jacob-induced displacement of LMO4 from CREB and subsequent transcriptional inactivation of CREB following activation of NMDAR by amyloid-β. The inventors thereby identified the compound (4-nitrophenyl)arsonic acid, also known as nitarsone (PubChem CID 66826). They next verified their finding in biochemical experiments followed by investigations in neuronal cultures exposed to amyloid-β. Final evidence for the efficacy of the treatment was obtained in animal experiments using transgenic Alzheimer's disease mouse models, as shown in the examples herein.

It was entirely surprising that targeting Jacob pharmacologically, as described herein, presents a highly beneficial approach to stop or slow Aβ pathology and CREB shutoff in the development of AD.

Hence, in embodiments of the invention the neurodegenerative disease is Alzheimer's Disease (AD).

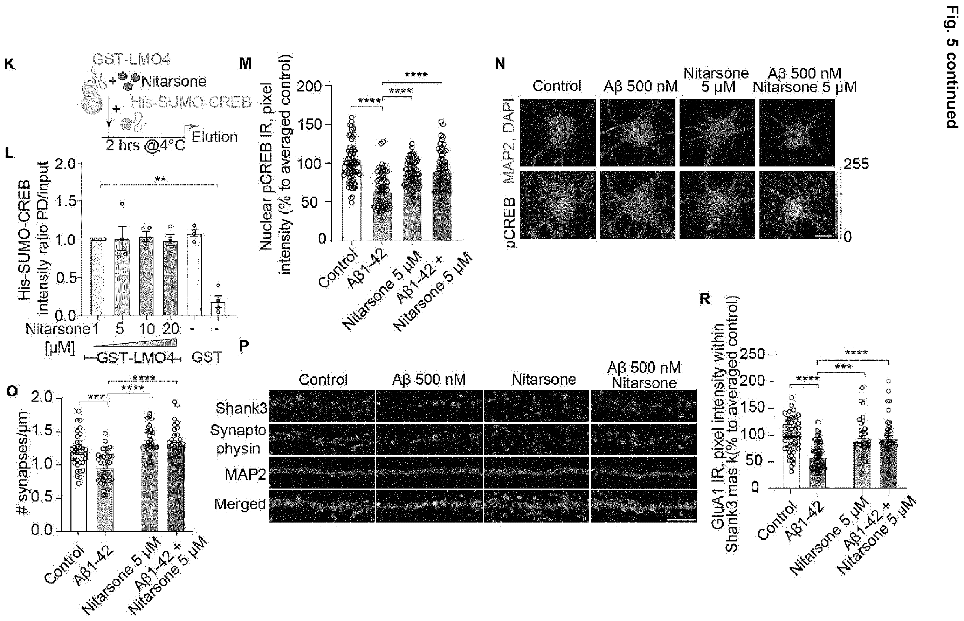

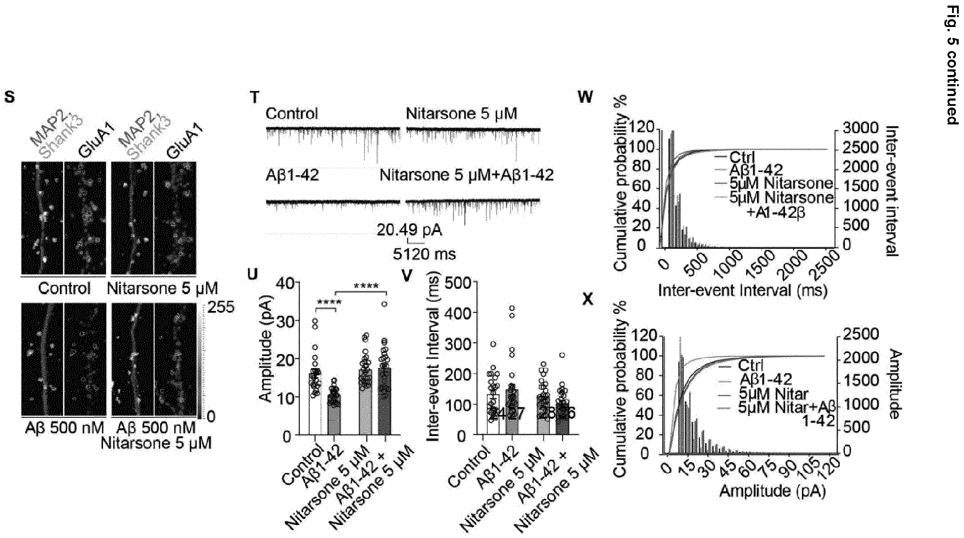

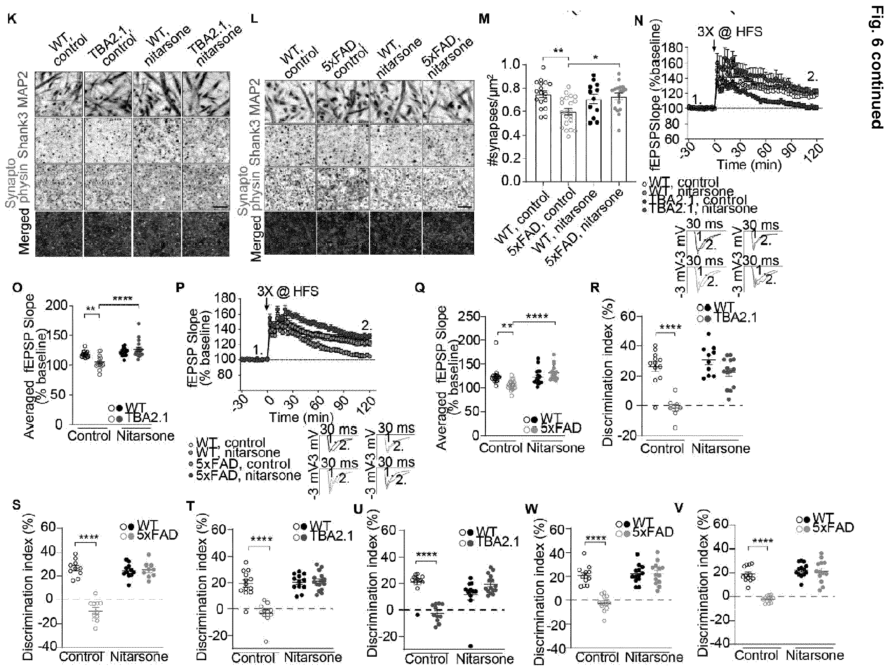

As evidenced by the Examples below, the treatment of subjects diagnosed or suspected of having a neurodegenerative disease, such as AD, PD, ALS or dementia, with nitarsone (or derivatives or salts thereof) opens up new and much more selective therapeutic avenues. For example, by directly targeting mechanisms underlying neurodegenerative dieses already at the onset, such as altered NMDAR-to-nucleus communication at the onset of AD. Interestingly, the inventors found that the improvement in spatial memory upon nitarsone treatment appeared to occur independently of Aβ plaque load and might be related to the extent of synapse loss, which is a more robust correlate of cognitive impairment in AD patients at an early stage than Aβ or neurofibrillary tangle deposition. Nitarsone selectively interrupts the interaction of Jacob but not of CREB to the LIM1 domain of LMO4 and competes with a 15 amino acid short peptide in Jacob that binds to LIM1. Moreover, the inventors identified two peptides within the LMO4 binding region of CREB. As shown in the Examples, the inventors observed that Amyloid Beta (Aβ)-induced synapse loss was completely prevented by nitarsone application (Fig. 5o, p) and that the concomitant downscaling of synaptic surface expression of GluA1 AMPA-receptors was also significantly attenuated in the presence of Nitarsone (Fig. 5r, s). As shown in the Examples below, the inventors surprisingly detected nitarsone treatment to restore synaptic plasticity and to improve hippocampus-dependent learning and memory despite the presence of manifest amyloid pathology. Nitarsone treatment reduces early neuronal cell loss in comparison to vehicle-treated AD models and the inventors observed prevention of neuronal loss in AD models treated with nitarsone. In addition, AD models treated with nitarsone displayed improved discrimination performance in comparison to vehicle treated models (Fig. 6q, r). This is particularly relevant, as human AD patients display impairments in object recognition tasks which essentially rely on proper synaptic function of CA1 neurons (Didic et al., 2013). As AD, PD and dementia are lethal neurodegenerative diseases starting usually at higher age, the inventors consider Nitarsone a reasonable therapeutic option to attenuate early synaptic dysfunction and cognitive decline and thereby to slow down disease progression. In embodiments the most promising therapeutic window in AD is right at the beginning of synaptic dysfunction, and in light of the present Examples Jacob is an attractive target for interventions to rescue or even restore synaptic plasticity.

Accordingly, the use of nitarsone or derivatives or salts thereof in the treatment of a neurodegenerative disease has the potential to prevent, ameliorate or reduce the symptoms and progression of the disease. Hence the invention relates in embodiments to the use of nitarsone, or derivatives or salts thereof in the treatment of a neurodegenerative disease, wherein symptoms and/or the progression of the disease is prevented, ameliorated, slowed down or reduced.

The inventors demonstrate in the examples herein the relevance of the identified and targeted mechanism for early synaptic failure in AD by targeting a crucial protein-protein interaction responsible for CREB shutoff with the small chemical compound nitarsone. Collectively, embodiments of the invention facilitate the manipulation of macromolecular protein transport mechanisms from NMDAR to the nucleus to stop or slow the disease progression at an early stage of AD. No other molecular mechanism for long-lasting transcriptional inactivation of CREB in neurons has been described yet and it is suggested by the findings of the inventors that this mechanism will also contribute to early synaptic dysfunction elicited by similar mechanisms in other slowly progressing neurodegenerative diseases.

Hence, in other embodiments of the invention the neurodegenerative disease is dementia.

In embodiments the invention relates to a compound nitarsone, or derivative or salt thereof for use in the treatment of dementia.

In other embodiments the neurodegenerative disease is Parkinson's Disease (PD). In embodiments the invention relates to a compound nitarsone, or derivative or salt thereof for use in the treatment of Parkinson's Disease.

In other embodiments the neurodegenerative disease is amyotrophic lateral sclerosis (ALS).

In embodiments the invention relates to a compound nitarsone, or derivative or salt thereof for use in the treatment of amyotrophic lateral sclerosis (ALS).

Further, in embodiments the present invention relates to a compound nitarsone, or derivative or salt thereof for use in the treatment of a neurodegenerative disease in a subject to prevent the transcriptional inactivation of CREB or wherein the transcriptional inactivation of CREB is prevented.

In embodiments the present invention relates to a compound nitarsone, or derivative or salt thereof for use in the treatment of a neurodegenerative disease in a subject to disrupt the molecular interaction between the protein Jacob and LMO4 or wherein the molecular interaction between the protein Jacob and LMO4 is interrupted.

In embodiments the present invention also relates to a method of treating a neurodegenerative disease in a subject by disrupting the molecular interaction between the protein Jacob and LIM domain only 4 (LMO4; a transcriptional co-activator of cAMP-response element binding protein (CREB)), comprising administering a therapeutically effective amount of a compound nitarsone or salt thereof.

The disruption of the molecular interaction between the protein Jacob and the transcriptional co-activator of cAMP-response element binding protein (CREB), prevents alterations in gene expression that cause progression of neurodegenerative diseases by transcriptional inactivation of CREB. Hence, the invention provides novel and improved means for treating neurodegenerative diseases characterized by disruption of synaptic transmission and/or related impairment in neuronal function, such as Alzheimer's disease (AD) or dementia.

Accordingly, the present invention provides new and surprising solutions for treating, reducing and/or preventing cognitive and/or neurological symptoms of a neurodegenerative disease, such as acute and/or chronic neurological and cognitive impairment. A shown in the examples herein nitarsone prevents the impairment of synaptic plasticity, synapse loss and neural loss as well as cognitive decline associated with neurodegenerative diseases, such as AD or dementia.

As is shown in more detail below, the present invention is based on the detailed knowledge of the molecular mechanism in which Jacob-driven complex disrupts CREB function by LMO4 displacement, and the objective of the present invention is to provide a chemical compound preventing this displacement, addressing in a novel, very specific way the problem of gene expression impairment linked to the synapse loss in AD and correlated memory loss. Surprisingly, the substance based on the chemical structure according to the invention could provide a new approach for the treatment of AD and related conditions.

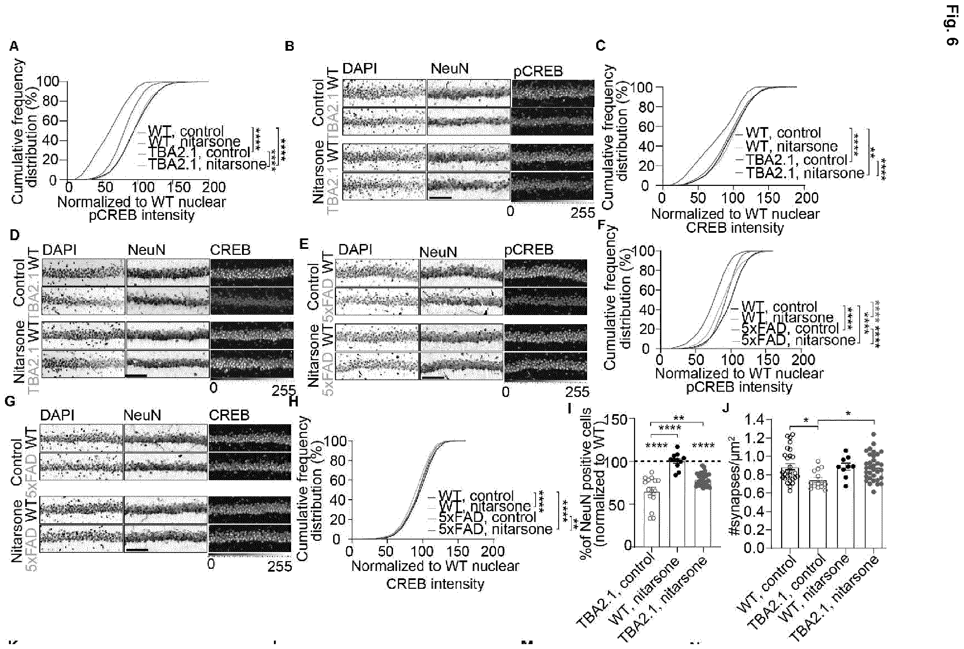

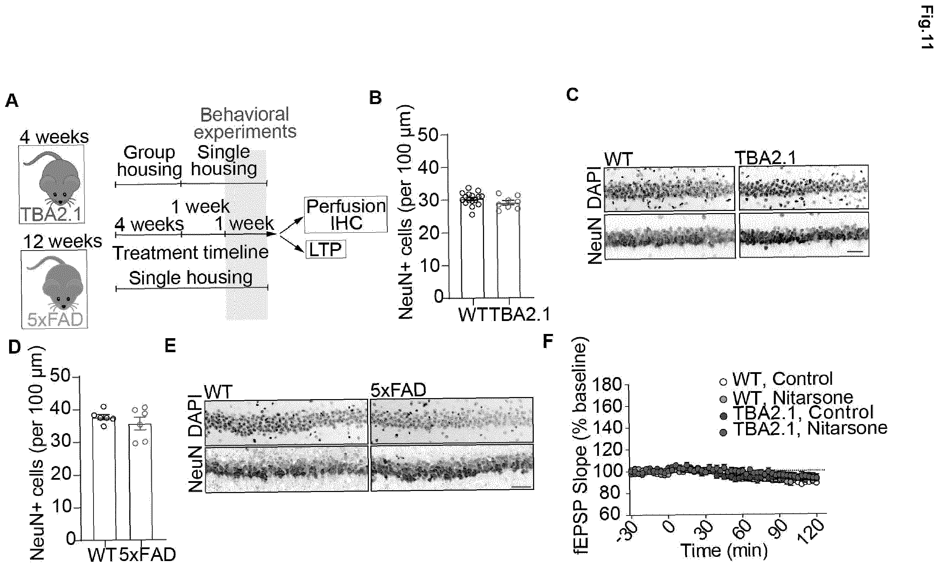

The inventors could reveal that administration of nitarsone effectively prevents CREB shutoff in the dorsal hippocampal CA1 brain region, whereby early neuronal cell loss can effectively be reduced and short-term memory can be improved (see Examples herein). In addition, the inventors found that nitarsone administration has the unexpected potential to reduce synapse loss and prevent neuronal cell loss in AD (see examples and e.g. Fig. 6j-m). Furthermore, the nitarsone mediated rescue of synaptic plasticity is important for the preservation of cognitive skills required for learning and memory in AD (see examples and e.g. Fig. 6n-q, Fig. 11f-g).

In addition, administration of nitarsone displayed improved discrimination performance in comparison to vehicle treated animal models (see e.g. Fig. 6q, r), which is highly relevant for human AD and dementia patients that usually display impairments in object recognition tasks, which essentially relies on proper synaptic function of CA1 neurons (Didic et al., 2013).

Accordingly, in embodiments of the invention the subject is showing one or more symptoms of a neurodegenerative disease, such as impaired memory, language, perceptual skills, attention, motor skills, orientation, problem solving and/or executive functional abilities.

In embodiments of the invention nitarsone, or derivatives or salts thereof may refer to a compound having one of the following Markush structures: 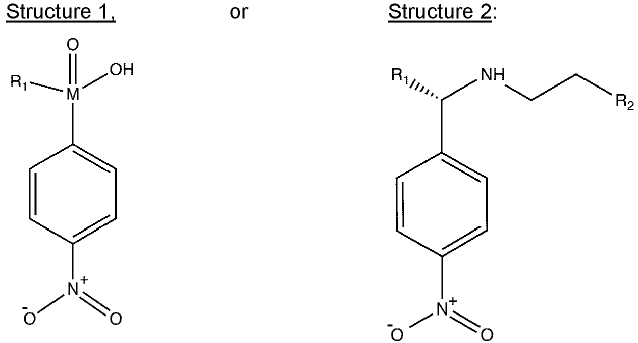 , Wherein in Structure 1 preferably
M is comprising As or Sb
and
R1 is comprising
   - -OH or
   - -OR3OH, wherein R3 is an alkyl chain comprising 1 to 5 alkyl groups, preferably 1-3, more preferably 1,
and wherein in structure 2 preferably
R1 is comprising
   - an alkyl chain comprising 1 to 5 alkyl groups, preferably 1-3, more preferably 1 or
   - -R3OH, wherein R3 is an alkyl chain comprising 1 to 5 alkyl groups, preferably 1-3, more preferably 1 or
   - -OH or
   - -NR4₂, wherein R4 is an alkyl group or hydrogen
and
R2 is comprising -OH or -SH.

In specific embodiments nitarsone may refer to the compound with the structural formula: 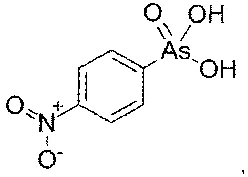 or salts thereof.

Accordingly, in embodiments the invention relates to a compound nitarsone, or derivatives or salts thereof for use in the treatment of a neurodegenerative disease in a subject, wherein nitarsone has the structural formula

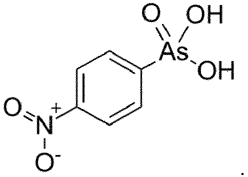

In embodiments the invention relates to a compound nitarsone, or derivative or salt thereof for use in the treatment of a neurodegenerative disease in a subject, wherein the compound nitarsone, or derivative or salt thereof has the structural formula: 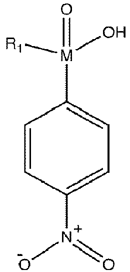 , Wherein M is comprising As or Sb and
R1 is comprising
- -OH or
- -OR3OH, wherein R3 is an alkyl chain comprising 1 to 5 alkyl groups, preferably 1-3, more preferably 1.

In further embodiments the invention relates to a compound nitarsone, or derivative or salt thereof for use in the treatment of a neurodegenerative disease in a subject, wherein the compound nitarsone, or derivative or salt thereof has the structural formula: 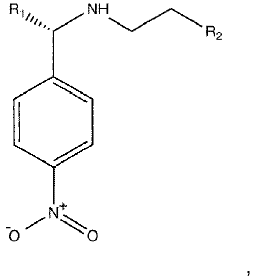 Wherein R1 is comprising
- an alkyl chain comprising 1 to 5 alkyl groups, preferably 1-3, more preferably 1 or
- -R3OH, wherein R3 is an alkyl chain comprising 1 to 5 alkyl groups, preferably 1-3, more preferably 1 or
- -OH or
- -NR4₂, wherein R4 is an alkyl group or hydrogen
and
R2 is comprising -OH or -SH.

In embodiments the compound nitarsone, or derivative or salt thereof may be selected from the group comprising (4-nitrophenyl)arsonic acid, (4-nitrophenyl)stibonic acid, hydroxymethyl hydrogen(4-nitrophenyl)arsonate and hydroxymethyl hydrogen(4-nitrophenyl)stibonate.

In some embodiments the present invention relates not only to nitarsone or salts thereof, but also to structural analogues or derivatives of nitarsone, which might be used in the treatment of neurodegenerative diseases according to the invention.

Accordingly, in embodiments the invention relates to structural analogues or derivatives of the compound nitarsone or salt thereof for use in the treatment of a neurodegenerative disease in a subject.

In some embodiments nitarsone, or derivatives or salts thereof may comprise or relate to substances selected from the group of nitarsone analogues or derivatives comprising (4-nitrophenyl)arsonic acid, (4-nitrophenyl)stibonic acid, hydroxymethyl hydrogen(4-nitrophenyl)arsonate and hydroxymethyl hydrogen(4-nitrophenyl)stibonate, having the following chemical structures: 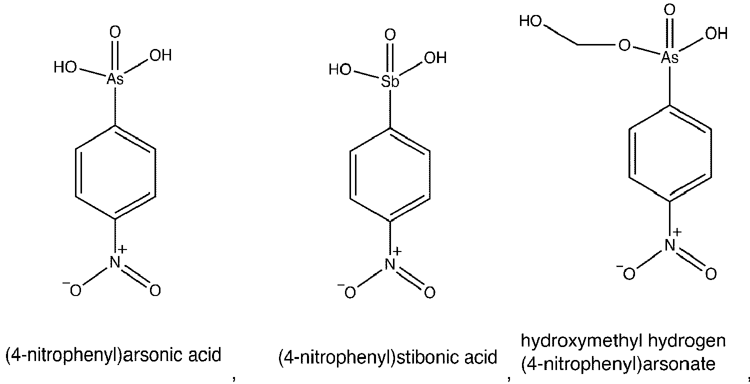 and

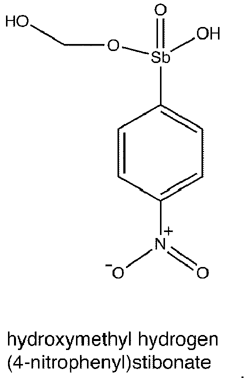

In some embodiments a structural analogue or derivative of nitarsone might be 2-{[1-(4-nitrophenyl)ethyl]amino}ethan-1-ol (ZINC37177221), also referred to as NPEAE, or analogues or derivatives thereof.

Hence, in certain embodiments the compound nitarsone, or derivative or salt thereof is 2-{[1-(4-nitrophenyl)ethyl]amino}ethan-1-ol (NPEAE), or derivatives or analogues thereof.

The inventors surprisingly found that also the nitrophenyl group of NPEAE and analogues or derivatives thereof fit into to the hydrophobic binding pocket of LIM1 and are considered to be able to prevent binding of Jacob to LIM1 of LMO4. In addition, NPEAE and its analogues or derivatives are soluble in water and have been predicted by LightBBB (Shaker et al. (2021)) to be able to cross the blood-brain-barrier.

In embodiments NPEAE, or analogues or derivatives of NPEAE may comprise or be selected from the group comprising (S)-2-((1-(4-nitrophenyl)ethyl)amino)ethan-1-ol, (S)-2-((hydroxyl(4-nitrophenyl)methyl)amino)ethan-1-ol, (R)-2-((amino(4-nitrophenyl)methyl)amino)ethan-1-ol, (R)-2-((2-hydroxyethyl)amino-2-(4-nitrophenyl)ethan-1-ol, (S)-2-((1-(4-nitrophenyl)amino)ethane-1-thiol, (S)-((2-mercaptoethyl)amino)(4-nitrophenyl)methanol, (R)-2-((amino(4-nitrophenyl)methyl)amino)ethane-1-thiol and (R)-2-((2-mercaptoethyl)amino)2-(4-nitrophenyl)ethan-1-ol, having the following chemical structures:

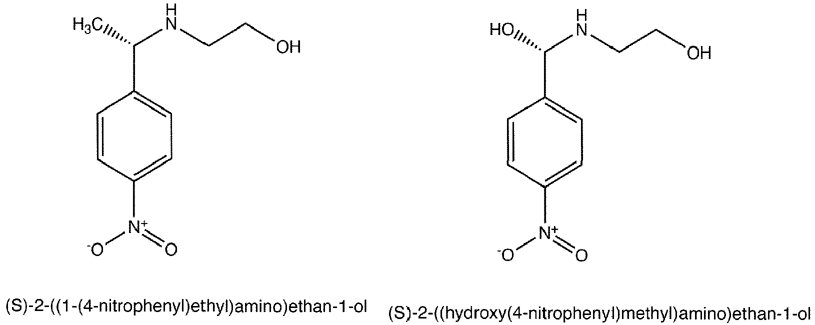

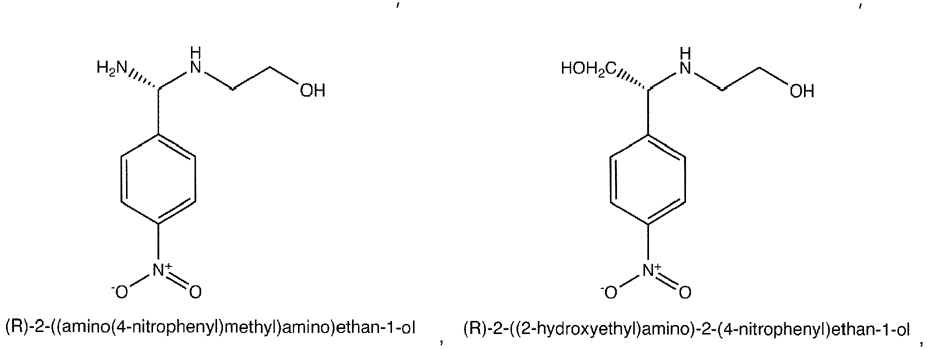

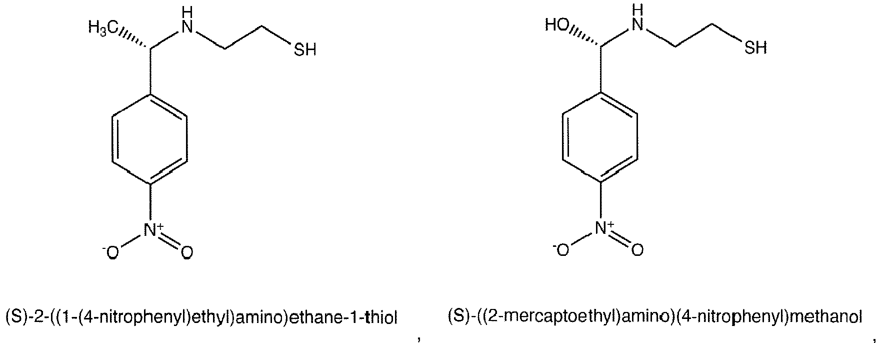

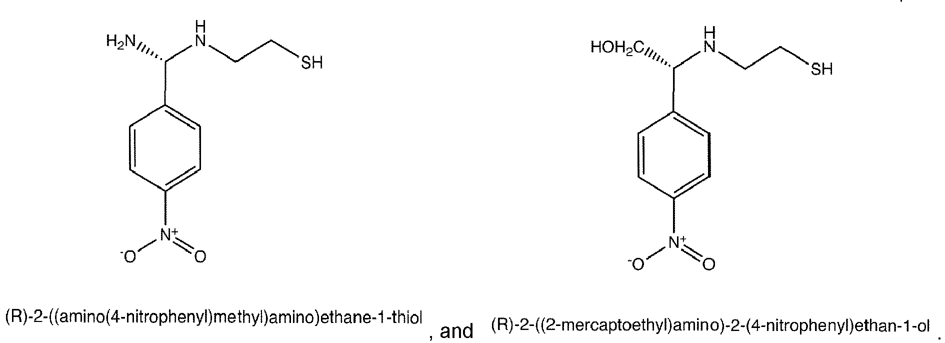

Accordingly, in embodiments the nitarsone, or derivative or salt thereof is selected from the group comprising (S)-2-((1-(4-nitrophenyl)ethyl)amino)ethan-1-ol, (S)-2-((hydroxyl(4-nitrophenyl)methyl)amino)ethan-1-ol, (R)-2-((amino(4-nitrophenyl)methyl)amino)ethan-1-ol, (R)-2-((2-hydroxyethyl)amino-2-(4-nitrophenyl)ethan-1-ol, (S)-2-((1-(4-nitrophenyl)amino)ethane-1-thiol, (S)-((2-mercaptoethyl)amino)(4-nitrophenyl)methanol, (R)-2-((amino(4-nitrophenyl)methyl)amino)ethane-1-thiol and (R)-2-((2-mercaptoethyl)amino)2-(4-nitrophenyl)ethan-1-ol.

The inventors surprisingly revealed by in silico modeling that the nitrophenyl group of NPEAE and structural analogues or derivatives thereof also fits into to the hydrophobic binding pocket of LIM1 thereby preventing the binding of Jacob to LIM1 of LMO4.

The invention also relates to a pharmaceutical composition comprising a compound nitarsone, or derivative or salt thereof for use in the treatment of a neurodegenerative disease in a subject wherein the pharmaceutical composition is provided in a liquid or in a solid form.

In another embodiment the compound nitarsone, or derivative or salt thereof is in solid form.

In embodiments the pharmaceutical composition is in solid form.

In embodiments the solid form is selected from the group comprising a tablet, an extended-release tablet, a coated tablet, a capsule, a dragee, a pill, a film, a lozenge and a powder.

In embodiments the pharmaceutical composition is in liquid form.

In another embodiment the compound nitarsone, or derivative or salt thereof is in liquid form. In embodiments the liquid form is selected from the group comprising a solution, an emulsion or a suspension.

In the context of the present invention other formulations of nitarsone or a salt thereof are also contemplated.

In embodiments the pharmaceutical composition is administered orally, transmucosally, intravenously or intramuscular.

The invention also relates to a pharmaceutical composition comprising a compound nitarsone, or derivative or salt thereof for use in the treatment of a neurodegenerative disease in a subject, wherein the composition is administered orally.

In embodiments, the composition is administered intravenously or intramuscular.

In embodiments, the composition is administered via the transmucosal route, such as intranasally, to the oral cavity, sub-lingually or buccally. In addition, a transmucosally, e.g., intranasally, applied nitarsone compound may more readily enter the brain avoiding first pass metabolism.

In embodiments the invention relates to the compound nitarsone, or derivative or salt thereof for the uses described herein, wherein the nitarsone, or derivative or salt thereof is present in liquid form at a concentration of 1 to 500 mg/mL, preferably between 1 and 400 mg/mL, more preferably between 1 and 300 mg/mL, preferably between 1 and 200 mg/mL, preferably between 5 and 100 mg/mL, between 1 to 200 mg/mL, preferably at a concentration of 5 to 100 mg/mL.

In embodiments, nitarsone, or derivative or salt thereof is present in liquid form at about 5 mg/mL, or 10, 15, 20, 25, 30, 35, 40, 45, 50, 55, 60, 65, 70, 75, 80, 85, 90, 95, 100, 110, 120, 130, 140, or at about 150 mg/mL. Ranges constructed form any given of the afore-mentioned values are also contemplated. In some embodiments the concentration of nitarsone, or derivative or salt thereof is dependent on or in correlation to the abundance of LMO4 and/or Jacob in one or more samples from the subject, such as blood, plasma, liquor or biopsy samples.

In embodiments the nitarsone, or derivative or salt thereof is comprised at the above concentrations within a pharmaceutical composition, wherein the pharmaceutical composition is in a liquid form.

In embodiments the invention relates to the compound nitarsone, or derivative or salt thereof for the uses described herein, wherein the nitarsone, or derivative or salt thereof is present in solid form at a concentration of 0.1 to 500 mg, preferably between 0.5 and 400 mg, more preferably between 1 and 300 mg, preferably between 1 and 200 mg, preferably between 5 and 100 mg, between 1 to 200 mg, preferably at a concentration of 5 to 100 mg.

In embodiments, the compound nitarsone, or derivative or salt thereof is present in solid form at about 5 mg, or 10, 15, 20, 25, 30, 35, 40, 45, 50, 55, 60, 65, 70, 75, 80, 85, 90, 95, 100, 110, 120, 130, 140, or at about 150 mg. Ranges constructed form any given of the afore-mentioned values are also contemplated.

In embodiments the nitarsone, or derivative or salt thereof is comprised at the above concentrations within a pharmaceutical composition, wherein the pharmaceutical composition is in a solid form.

In embodiments, the salt comprises stoichiometric and/or non-stoichiometric salts and/or hydrates of the chemical substances according to nitarsone, whereby the salt is preferably described as:
Nitarsone · n HX · m H₂O,
wherein n and m = 0 - 5, and n and m can be the same or different, and HX is an acid, selected preferably from lactic acid, gluconic acid, maleic acid or saccharic acid.

In some embodiments, other acids may be employed for the nitarsone salt formation.

In one embodiment, the nitarsone salt has solubility in water of at least 10%, preferably > 20%, or more preferably >30% weight per volume (w/v). In some embodiments the solubility of nitarsone, or derivative or salt thereof is between 1 and 10 mg/mL at room temperature (25 °C), in some embodiments the solubility is between 13.44 mg/mL (1.344%) and 3.83 mg/mL (0.383%) at room temperature. A high solubility enables higher concentrations of the compound to be administered in smaller volumes, thereby further enhancing administration.

In embodiments, the nitarsone, or derivative or salt thereof is administered as a 0.1 to 50% weight per volume (w/v) solution at an amount of 0.5 to 500 milliliters in each administration event, at least one times daily, over one or multiple days. In embodiments, the nitarsone, or derivative or salt thereof is administered as a 0.5%, 1%, 1.25%, 1.5%, 1.75%, 1.76%, 2%, 2.25 %, 2.5%, 2.755, 3%, 4%, 5%, 7.5%, 10%, 15.5%, 15%, 20%, 25 %, 30%, 35%, 40%, 45% or 50% weight per volume (w/v) solution or as a solution with a concentration between 0.5%, and 50%, between 0.5 and 20%, between 0.5 and 10 %, between 1 and 10%, between 1 and 5 %, between 1 and 2.5% weight per volume (w/v).

In embodiments, the compound nitarsone, or derivative or salt thereof is administered at an amount of 1 to 500 mg in each administration event, at least one times daily, over one or multiple days.

In embodiments, the compound nitarsone, or derivative or salt thereof is administered at an amount between 0.001 and 100 mg/kg body weight of the patient or subject. In embodiments, the compound nitarsone, or derivative or salt thereof is administered at an amount between 0.01 and 50 mg/kg body weight, between 1 and 25 mg/kg body weight of the patient or subject. In embodiments, the compound nitarsone, or derivative or salt thereof is administered at an amount of 0.001 mg/kg body, 0.01 mg/kg body weight, weight, 0.1 mg/kg body weight, 0.25 mg/kg body weight, 0.5 mg/kg body weight, 1 mg/kg body weight, 1.5 mg/kg body weight, 2 mg/kg body weight, 2.5 mg/kg body weight, 3 mg/kg body weight, 4 mg/kg body weight, 5 mg/kg body weight, 6, 7, 8, 9, 10, 11, 12, 13, 14, 15, 16, 17, 18, 19, 20, 25, 30, 35, 40, 45, 50, 60, 65, 70, 75, 80, 85, 90, 95, 100, 250, 200, 250, 300, 350, 400, or 500 mg/kg body weight of the patient or subject.

In embodiments the subject is older than 15 years of age. In embodiments the subject is older than 30 years of age. In some embodiments the subject is an elderly subject. In embodiments the elderly subject is older than 40 years of age, or even older than 50 years of age. In some embodiments the elderly subject is older than 60 years of age.

In embodiments the compound nitarsone, or derivative or salt thereof is comprised within a pharmaceutical composition when administrated to a subject.

In embodiments the pharmaceutical composition for use according to the invention is administered at least 1 time per day. In embodiments the pharmaceutical composition for use according to the invention is administered at least once a week, at least twice a week, every other day, 1, 2, 3, 4, 5 or even 6 times per day. In embodiments the pharmaceutical composition for use according to the invention is administered at least 1 time per day, over one or more days. In embodiments the pharmaceutical composition for use according to the invention is administered at least one time per day for more than one consecutive days. In embodiments the pharmaceutical composition for use according to the invention is administered over 1, 2, 3, 4, 5, 6, 7, 8, 9, 10, 14, 21, 28, 30, 49, 56, 60, 90, 120, 365 days or even longer. In embodiments the pharmaceutical composition for use according to the invention is administered over at least 1, 2, 3, 4, 5, 6, 7, 8, 9, 10, 11, 12, 13, 14, 15, 20, 24, 25, 30, 36, 40, 42, 50, 58, 60, 120, 240, 360, 480 consecutive months or even longer.

In some embodiments, the liquid formulations are configured for any one or more of the above administration modes. A skilled person is aware of technical means employed in configuring compositions for specific modes of administration. For example, a composition configured for nasal administration may be formulated, packaged, or prepared in a different manner from compositions prepared for oral administration.

The invention also relates to a pharmaceutical composition comprising a compound nitarsone, or derivative or salt thereof for use in the treatment of a neurodegenerative disease in a subject, wherein the decline of cognitive function in a subject is prevented or slowed down.

Accordingly, in embodiments, the invention relates to the compound nitarsone, or derivative or salt thereof for use according to the present invention, wherein the subject exhibits cognitive impairment with clinical symptoms of dementia and/or AD.

In another embodiments the invention also provides means for treating and/or preventing deficits or decline in cognition and memory associated with a neurodegenerative disease in a subject.

The invention therefore also relates to a method for treating a neurodegenerative disease associated with cognitive and/or neurological impairment in a subject, the method comprising administering a therapeutically effective amount of nitarsone, or derivative or salt thereof to a subject.

Accordingly in one embodiment the invention relates to a method of improving cognitive function in a subject suspected or diagnosed with a neurodegenerative disease, comprising administering a therapeutically effective amount of a compound nitarsone, or derivative or salt thereof to said subject. The invention also relates to a pharmaceutical composition comprising a compound nitarsone, or derivative or salt thereof for use in the treatment of a confirmed or suspected neurodegenerative disease in a subject, wherein cognitive function in a subject is improved or preserved.

In one embodiment the invention relates to a method of preventing or slowing the decline of cognitive function in a subject suspected or diagnosed with a neurodegenerative disease, comprising administering a therapeutically effective amount of a compound nitarsone, or derivative or salt thereof to said subject.

In embodiments the invention relates to the compound nitarsone, or derivative or salt thereof for use in improving short-term memory in a subject suspected or diagnosed with a neurodegenerative disease.

The instant disclosure also includes kits, packages and multi-container units containing the herein described pharmaceutical compositions, active ingredients, and/or means for administering the same for use in the prevention and treatment of diseases and other conditions in mammalian subjects.

Embodiments and features of the invention described with respect to the pharmaceutical composition, the substance and salts thereof, and various methods described herein, are considered to be disclosed with respect to each and every other aspect of the disclosure, such that features characterizing the methods, may be employed to characterize the composition, or the substance and vice-versa. The various aspects of the invention are unified by, benefit from, are based on and/or are linked by the common and surprising finding of the beneficial and curative effect of the use of nitarsone in the treatment of neurodegenerative diseases.

### DETAILED DESCRIPTION OF THE INVENTION

All cited documents of the patent and non-patent literature are hereby incorporated by reference in their entirety.

The invention is directed to a compound nitarsone, or derivative or salt thereof for use in the treatment of a neurodegenerative disease, such as Alzheimer's disease (AD), dementia, Parkinson's disease (PD) or amyotrophic lateral sclerosis (ALS). In another aspect the present invention relates to a pharmaceutical composition comprising the compound, or derivative or salt thereof according to the invention.

"Neurodegenerative diseases" are caused by the progressive loss of structure or function of neurons, in the process known as neurodegeneration. Such neuronal damage may ultimately involve cell death. Neurodegenerative diseases include amyotrophic lateral sclerosis (ALS), multiple sclerosis (MS), Parkinson's disease (PD), Alzheimer's disease (AD), Huntington's disease, multiple system atrophy, and prion diseases. As neurodegenerative diseases might cause dementia, in the context of the present invention also dementia might be referred to as a neurodegenerative disease or may be comprised within the group of "neurodegenerative diseases".

"Alzheimer's disease (AD)" is a neurodegenerative disease that usually starts slowly and progressively worsens. It is the cause of 60-70% of cases of dementia. The most common early symptom is difficulty in remembering recent events. As the disease advances, symptoms are worsening. AD symptoms can be categorized into eight intellectual domains that are most commonly impaired in AD-memory, language, perceptual skills, attention, motor skills, orientation, problem solving and executive functional abilities, as listed in the fourth text revision of the DSM (DSM-IV-TR). The disease progression is also accompanied by the decline or loss of bodily functions, which ultimately leads to death. Although the speed of progression can vary, the life expectancy following diagnosis is generally significantly decreased. By today, no treatments are available to stop or reverse AD progression, the only available therapies aim to temporarily improve some symptoms. Alzheimer's disease may be characterized by loss of neurons and synapses in the cerebral cortex and certain subcortical regions. This loss results in gross atrophy of the affected regions, including degeneration in the temporal lobe and parietal lobe, and parts of the frontal cortex and cingulate gyrus. Degeneration is also present in brainstem nuclei particularly the locus coeruleus in the pons.

Alzheimer's disease has been identified as a protein misfolding disease, a proteopathy, caused by the accumulation of abnormally folded amyloid beta protein into amyloid plaques, and/or tau protein into neurofibrillary tangles in the brain. Plaques can be made up of small peptides, 39-43 amino acids in length, called amyloid beta (Aβ). Amyloid beta is a fragment from the larger amyloid-beta precursor protein (APP) a transmembrane protein that penetrates the neuron's membrane. APP is critical to neuron growth, survival, and post-injury repair. In Alzheimer's disease, gamma secretase and beta secretase can act together in a proteolytic process which causes APP to be divided into smaller fragments. One of these fragments gives rise to fibrils of amyloid beta, which then form clumps that deposit outside neurons in dense formations known as amyloid plaques. Alzheimer's disease can also be considered a tauopathy due to abnormal aggregation of the tau protein. Every neuron has a cytoskeleton, an internal support structure partly made up of structures called microtubules. These microtubules act like tracks, guiding nutrients and molecules from the body of the cell to the ends of the axon and back. The protein tau stabilizes the microtubules when phosphorylated. In Alzheimer's disease, tau undergoes chemical changes, becoming hyperphosphorylated; it then begins to pair with other threads, creating neurofibrillary tangles and disintegrating the neuron's transport system. Pathogenic tau can also cause neuronal death through transposable element dysregulation.

"Dementia" is a condition comprising a variety of related symptoms, comprising progressive impairments in memory, thinking, and behavior, which negatively impact a person's ability to function and carry out everyday activities. Besides memory impairment and the disruption in thought patterns, the most common symptoms comprise emotional problems, difficulties with language, and decreased motivation. Several diseases and injuries to the brain, such as a stroke, can give rise to dementia. However, the most common cause is Alzheimer's disease, a neurodegenerative disorder. Vascular dementia is the second most common type of dementia and is caused by reduced blood supply to the brain due to diseased blood vessels. Mixed dementia is a condition in which a person suffers from more than one type of dementia, whereby Alzheimer's disease and vascular dementia are the most common types. Frontotemporal dementias (FTDs) are characterized by drastic personality changes and language difficulties. In all FTDs, the person has a relatively early social withdrawal and early lack of insight. A frontotemporal dementia associated with amyotrophic lateral sclerosis (ALS) known as (FTD-ALS) includes the symptoms of FTD (behavior, language and movement problems) co-occurring with amyotrophic lateral sclerosis (loss of motor neurons). Two FTD-related disorders are progressive supranuclear palsy (also classed as a Parkinson-plus syndrome), and corticobasal degeneration. Such disorders are tau-associated. There is no known cure for dementia to date. In the context of the present invention dementia might be referred to as a neurodegenerative disease or may be comprised within the group of neurodegenerative diseases.

The term "subject" or "patient may be in the context of the invention preferably be a mammal, more preferably a human. Herein the terms "subject" or "patient may be used interchangeably. In the context of the present invention a subject or patient has preferably been diagnosed with a neurodegenerative disease or may be suspected of having a neurodegenerative disease and/or may be showing early signs of a neurodegenerative disease. In embodiments the treatment according to the invention can prevent or slow down the progress of a neurodegenerative disease in a subject, hence subjects with any stage of a neurodegenerative disease may in embodiments benefit from receiving a treatment with nitarsone. A shown in the Examples herein nitarsone prevents the impairment of synaptic plasticity, synapse and neural loss as well as cognitive decline associated with AD.

"Nitarsone" (IUPAC (4-Nitrophenyl)arsonic acid or Nitrobenzenearsonic acid) is an organoarsenic compound with the CAS Registry Number 98-72-6; PubChem CID 66826; and the chemical formula C₆H₆AsNO₅:

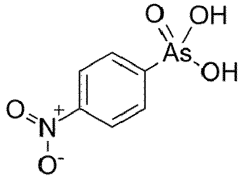

In the context of the present invention nitarsone, or derivatives or salts thereof or structural analogues of nitarsone might be used according to the invention. In the context of embodiments of the present invention a structural analogue or derivative of nitarsone might be 2-{[1-(4-nitrophenyl)ethyl]amino}ethan-1-ol (ZINC37177221), NPEAE or derivatives or structural analogues thereof. The inventors surprisingly found that also the nitrophenyl group of NPEAE and structural analogues or derivatives thereof also fit into to the hydrophobic binding pocket of LIM1 thereby preventing the binding of Jacob to LIM1 of LMO4.

The present invention encompasses both treatment and prophylactic treatment of a subject. A "prophylactic" treatment is a treatment administered to a subject who does not exhibit signs of a disease or exhibits only early signs for the purpose of decreasing the risk of developing pathology.

The present invention relates further to pharmaceutically acceptable salts of the compounds described herein. The term "pharmaceutically acceptable salt" refers to salts or esters prepared by conventional means that include basic salts of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. Any chemical compound recited in this specification may alternatively be administered as a pharmaceutically acceptable salt thereof.

"Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

A dotted line in the position of a double bond represents an optional double bond, which may be present or absent. Herein a Markush structure is understood as a representation of chemical structures to represent a group of related chemical compounds and/or analogues and/or derivatives, wherein "R" stands for a side chain with a varying structure and "M" stands for an atom with varying nature.

Protected derivatives of the disclosed compound also are contemplated. A variety of suitable protecting groups for use with the disclosed compounds are disclosed in Greene and Wuts Protective Groups in Organic Synthesis; 3rd Ed.; John Wiley & Sons, New York, 1999. In general, protecting groups are removed under conditions which will not affect the remaining portion of the molecule. These methods are well known in the art and include acid hydrolysis, hydrogenolysis and the like.

It is understood that substituents and substitution patterns of the compounds described herein can be selected by one of ordinary skill in the art to provide compounds that are chemically stable and that can be readily synthesized by techniques known in the art and further by the methods set forth in this disclosure.

Another aspect of the disclosure includes pharmaceutical compositions prepared for administration to a subject and which include a therapeutically effective amount of one or more of the compounds disclosed herein. In certain embodiments, the pharmaceutical compositions are useful for treating Alzheimer's disease, dementia or Parkinson's disease. The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, antiinflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The pharmaceutical compositions can be administered to subjects by a variety of mucosal administration modes, including by oral, rectal, intraocular, intranasal, intrapulmonary, or transdermal delivery, or by topical delivery to other surfaces. Optionally, the compositions can be administered by non-mucosal routes, including by intramuscular, intraocular, subcutaneous, intravenous, intra-arterial, intra-articular, intraperitoneal, intrathecal, intracerebroventricular, or parenteral routes.

The compositions of the disclosure can alternatively contain as pharmaceutically acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of and "administering a" compound should be understood to mean providing a compound, a prodrug of a compound, or a pharmaceutical composition as described herein. The compound or composition can be administered by another person to the subject (e.g., intravenously) or it can be self-administered by the subject (e.g., tablets).

Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The present invention also relates to a method of treatment of subjects suffering from the various medical conditions disclosed herein. The method of treatment comprises preferably the administration of a therapeutically effective amount of a compound disclosed herein to a subject in need thereof.

A "therapeutically effective amount" refers to a quantity of a specified agent sufficient to achieve a desired effect in a subject being treated with that agent. For example, this may be the amount of a compound disclosed herein useful in treating a disease of the thyroid in a subject. The therapeutically effective amount or diagnostically effective amount of an agent will be dependent on the subject being treated, the severity of the affliction, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects. A non-limiting range for a therapeutically effective amount of a compound and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

The compounds and compositions according to the invention may, if desired, be presented in a pack, vessel, vial or dispenser device, such as an EMA or FDA approved kit, which may contain dosage forms containing the active ingredient. The pack, vessel, vial or dispenser device may be accompanied by instructions for administration. The pack, vessel, vial or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Such notice, for example, may be of the labeling approved by the EMA or FDA for prescription drugs or of an approved product insert.

### FIGURES

The invention is further described by the following figures. These are not intended to limit the scope of the invention but represent preferred embodiments of aspects of the invention provided for greater illustration of the invention described herein.

### Brief description of the figures:

Figure 1: Jacob-regulated CREB phosphorylation decrease correlates with neuronal loss in an animal model of Alzheimer's disease and in Alzheimer's disease patients.
Figure 2: Jacob directly associates with CREB and LMO4.
Figure 3: Jacob displaces LMO4 from CREB.
Figure 4: CREB shutoff essentially requires Jacob binding to PP1.
Figure 5: Nitarsone disrupts LIM1-Jacob binding and rescues Aβ₁₋₄₂-induced CREB shutoff, synaptic AMPAR loss, and mEPSC amplitude impairment.
Figure 6: that treatment with Nitarsone rescues AD-related phenotype in TBA2.1 and 5xFAD mice.
Figure 7: Jacob-regulated CREB phosphorylation decreases in an animal model of Alzheimer's disease and in Alzheimer's disease patients.
Figure 8: Jacob-CREB interaction, mapping of the binding interfaces between Jacob and CREB.
Figure 9: Confirmation of Jacob-CREB interaction, mapping of the binding interfaces between Jacob and CREB.
Figure 10: Nitarsone disrupts Jacob-LMO4 interaction and rescues acute CREB shutoff.
Figure 11: Treatment with Nitarsone rescues AD-related phenotype in TBA2.1 and 5xFAD mice.
Figure 12: Graphic representation of scientific principle behind embodiments of the invention. (1) NMDAr-dependent synaptic signals lead to Jacob-dependent import of kinase, CREB activation, and subsequent expression of pro-survival genes.

### Detailed description of the figures:

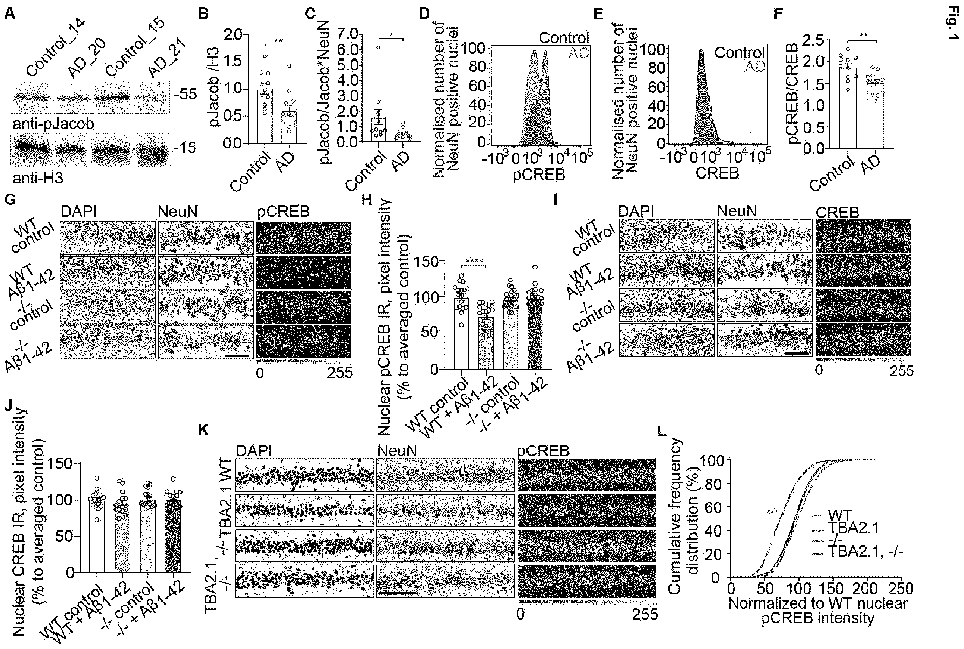

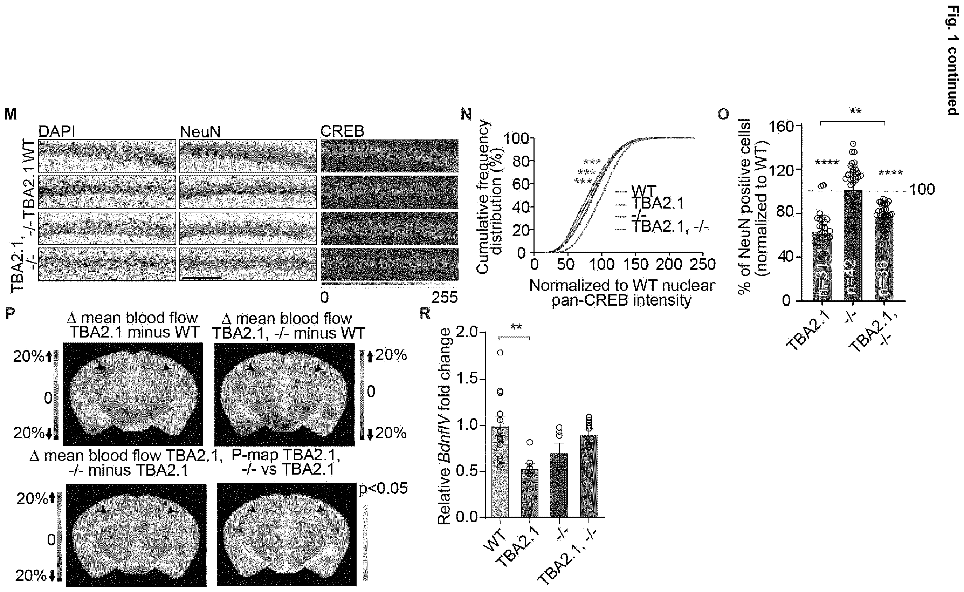

**Figure 1****:** The figure illustrates the molecular functional basis of the use of nitarsone according to embodiments of the invention. The Figure shows the results of the experiments described in the Examples herein, wherein the inventors found out that Jacob-regulated CREB phosphorylation decrease correlates with neuronal loss in an animal model of Alzheimer's disease and in Alzheimer's disease patients. **(A, B)** Total pJacob protein levels are significantly reduced in brain samples from Alzheimer's disease patients as compared to the control group. **(C)** pJacob/panJacob corrected by NeuN levels is decreased in brain samples from AD patients as compared to the control group. All samples are normalized to Histone3 (H3). N=11-12 different subjects. **(D-F)** FACS measurements revealed significantly decreased pCREB, but not CREB, immunoreactivity of neuronal nuclei in AD patients as compared to the control group. **(D, E)** Frequency distribution plot of neuronal nuclei immunoreactivity **of (D)** pCREB and **(E)** CREB. **(F)** pCREB/CREB ratio in neuronal nuclei. N=11-12 different subjects. **(G-J)** Acute (1h) Aβ₁₋₄₂ treatment does not induce CREB shutoff in organotypic hippocampal slices from Jacob knockout (-/-) mice. Representative confocal images of slices immunolabeled against **(G)** pCREB or **(I)** panCREB, co-labeled with NeuN and DAPI. Bar plots of **(H)** pCREB, N=2320-3669 nuclei from 16-22 slices, and **(J)** N=1177-2079 nuclei from 11-14 slices. **(K, L)** The quantification of pCREB intensity in NeuN-positive cells revealed a statistically significant decrease in pCREB immunoreactivity in TBA2.1 but not in double transgenic animals (TBA2.1, -/-). **(L)** Data represented as cumulative frequency distribution. N=655-1232 nuclei from 7-9 animals. **(K)** Representative confocal images of CA1 cryosections from 13 weeks old mice stained for NeuN, DAPI and pCREB. Scale bar: 100 µm. (M, **N)** The quantification of CREB intensity in NeuN positive cells revealed a statistically significant decrease in CREB immunoreactivity in Jacob/*Nsmf* knockout (-/-) and TBA2.1 Jacob/*Nsmf* knockout (TBA2.1, -/-) mice. **(M)** Representative confocal images of CA1 cryosections from 13 weeks old mice stained for NeuN, DAPI and CREB. Scale bar: 100 µm. **(O)** knockout data represented as cumulative frequency distribution. N=1668-3073 nuclei from 7-9 animals. **(O)** The TBA2.1 Jacob/*Nsmf* knockout (TBA2.1, -/-) mice display significantly lower degree of neuronal loss compared to TBA2.1 mice. The number of NeuN positive cells was normalized to WT group. N=31-42 CA1 images analyzed from 9-11 animals per genotype. **(P)** Significant changes in cerebral blood flow between TBA2.1 and WT, TBA2.1, -/- and WT, and TBA2.1 and TBA2.1, -/- as determined by ⁹⁹mTc-HMPAO SPECT measurements. Difference images overlay over a reference MR for comparison with TBA2.1 mice as described on panel labeling. Bregma -2.5. The statistically significant differences between TBA2.1 and double transgenic animal were detected in dorsal CA1. N=10 animals. (p<0,05) by two-tailed Student t-test. **(R)** Jacob knockout rescues decrease in the *BdnflV* gene transcription. Bar plot of mean *BdnflV* transcript levels in hippocampal homogenates normalized to β-actin as a reference gene. N=5-10 hippocampi. **(G, I, K, M)** Lookup table indicates the pixel intensities from 0 to 255. *p<0.05, **p<0.01, ***p<0.001, ****p<0.0001 by **(B, C, F)** two-tailed Student t-test or **(H, J, L, N, O, R)** two-way ANOVA followed by Bonferroni's multiple comparisons test. All data are represented as mean ± SEM.

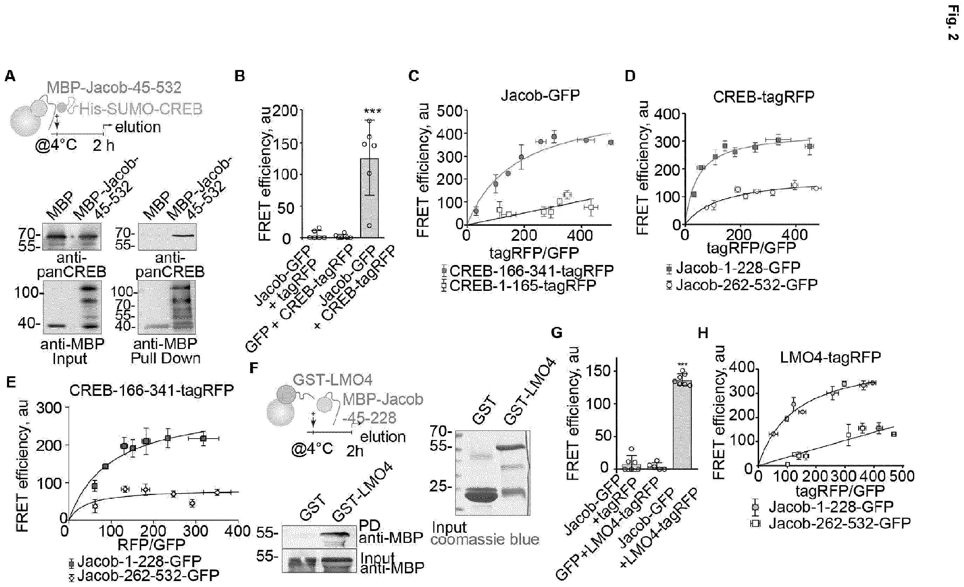

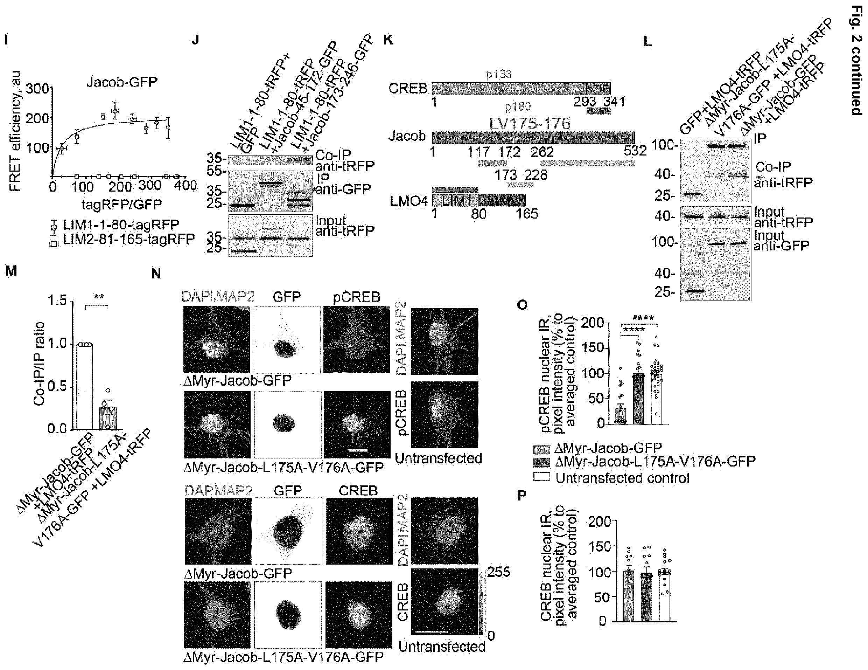

**Figure 2****:** The figure illustrates the molecular functional basis of the use of nitarsone according to embodiments of the invention. The Figure illustrates the results of the experiments described in the Examples herein, wherein the inventors found out that Jacob directly associates with CREB and LMO4. **(A)** Pull-down assay confirms a direct interaction of MBP-Jacob-45-532 and His-SUMO-CREB. **(B)** FRET measurements show association between CREB-tagRFP and Jacob-GFP. N=5 5 independent experiments measured in triplicates. **(C)** The C-terminus (CREB-166-341-tagRFP) but not the N-terminus (CREB-1-165-tagRFP) of CREB closely associates with Jacob-GFP in FRET saturation experiments. **(D)** Both N- (Jacob-1-228-GFP) and C-termini (Jacob-262-532-GFP) of Jacob are in close proximity to CREB-tagRFP, however, the Jacob-1-228-GFP association with CREB is significantly stronger. **(E)** The C-terminus of CREB (CREB-166-341-tagRFP) associates with both N- (Jacob-1-228-GFP) and C-termini (Jacob-262-532-GFP) of Jacob. However, association with N-terminus of Jacob-1-228-GFP is much stronger compared to its C-terminus. **(B-E)** FRET saturation experiments were performed in the HEK293T cell suspension. FRET efficiency is given in arbitrary units n= 5-6 independent experiments. **(F)** The GST-LMO4 fusion protein but not control GST pulls down recombinant MBP-Jacob-45-228. **(G)** FRET experiments revealed that Jacob-GFP interacts with LMO4-tagRFP. **(H, I)** FRET saturation experiments indicating the association of Jacob-1-228-GFP with LIM-1-80-tagRFP. (**G-I)** FRET efficiency is given in arbitrary units as a mean of N=6 independent experiments measured in triplicates. **(J)** Co-immunoprecipitation experiments to map the bonding region of Jacob to the LIM1 domain of LMO4 reviled the association with 179-246 aa of Jacob, but not with Jacob-45-172-GFP (CREB-binding region). **(K)** Schematic representations of domain organization and binding interfaces between CREB, Jacob, and LMO4. Depicted are full length human CREB protein (hCREB, top of scheme, SEQ ID NO 3), full length human Jacob (SEQ ID NO 1; full length hJacob and full length LMO4 (SEQ ID NO 2). Jacob binds to the bZIP domain of CREB (indicated by bar below CREB). The box indicates a stronger interaction of the Jacob N-terminus, the shaded box indicates a weaker association of the C-terminus. The LIM1 domain of LMO4 binds to 173-228 aa of Jacob (indicated by bar below Jakob). Leucin-valin motiv (LV) is crucial for Jacob-LMO4 interaction. **(L, M)** Heterologous co-immunoprecipitation experiments between LMO4-tagRFP and nuclear ΔMyr-Jacob-GFP, ΔMyr-Jacob-L175A-V176A-GFP or GFP overexpressed in HEK293T cells revealed decreased association of ΔMyr-Jacob-L175A-V176A-GFP with LMO4 compared to ΔMyr-Jacob-GFP. N=4 independent experiments. **(N-P)** A Jacob-LMO4-binding mutant expressed in the nucleus does not induce CREB shutoff. **(N, O)** Representative confocal images of hippocampal neurons transfected with ΔMyr-Jacob-GFP (Jacob targeted to the nucleus) or ΔMyr-Jacob-L175A-V176A-GFP. Scale bar: 10 µm. Lookup indicates the pixel intensities from 0 to 255. **(P)** The mean of nuclear pCREB immunoreactivity in Jacob-expressing neurons was normalized to un-transfected control. N - the number of neuronal nuclei analyzed from two independent cell cultures. **p<0.01, ***p<0.001, ****p<0.0001 by **(M)** one-sample t-test or one-way ANOVA followed by **(B, G)** Bonferroni's or **(O, P)** Tukey's multiple comparisons test. All data are represented as mean ± SEM.

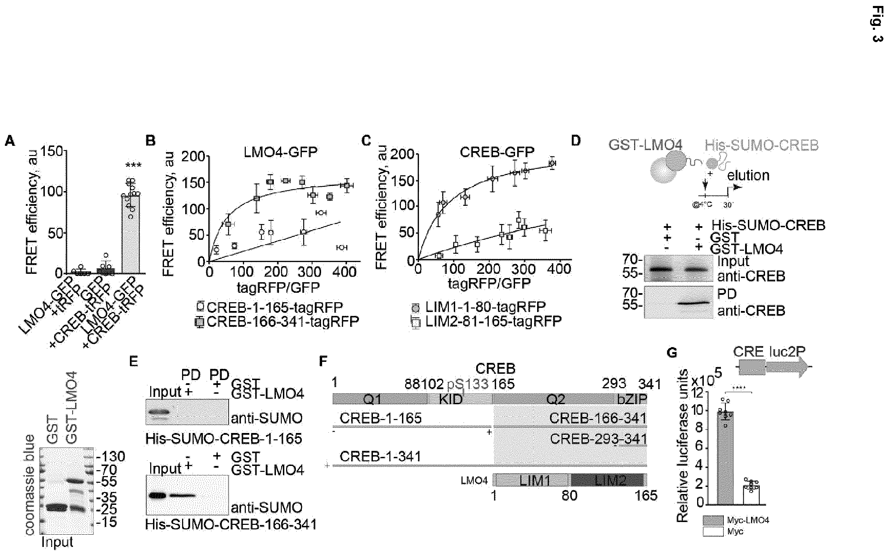

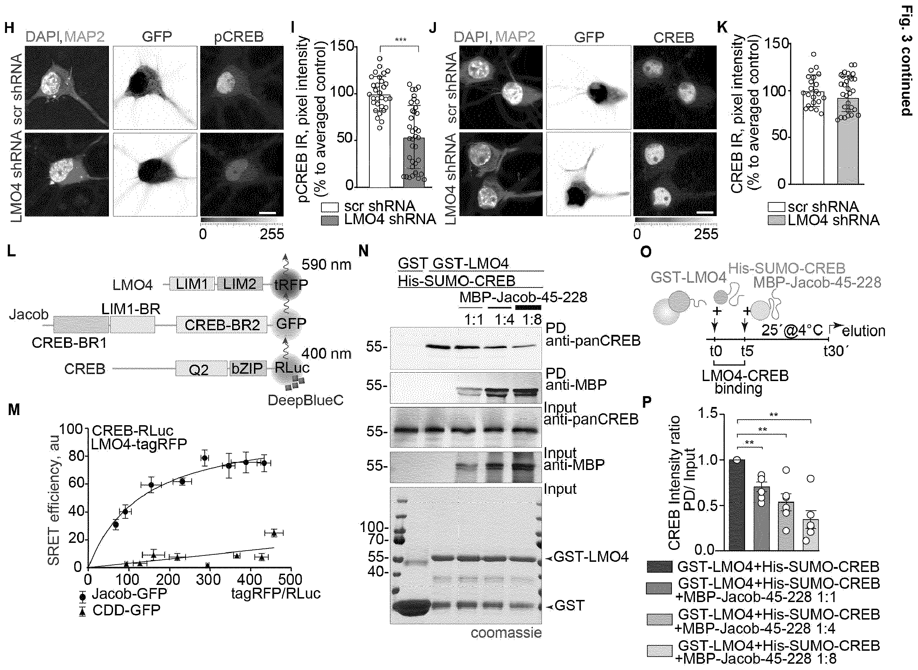

**Figure 3****:** The figure illustrates the molecular functional basis of the use of nitarsone according to embodiments of the invention. The Figure illustrates the results of the experiments described in the Examples herein, wherein the inventors found out that Jacob displaces LMO4 from CREB. **(A)** FRET measurements indicate a tight association between LMO4-GFP and CREB-tagRFP. N=6 independent experiments measured in triplicates. **(B, C)** FRET saturation experiments with **(B)** LMO4-GFP and CREB-1-165-tagRFP or **(C)** CREB-166-341-tagRFP and CREB-GFP and LIM1-1-80-tagRFP or LIM2-81-165-tagRFP revealed the association between LIM1 domain of LMO4 and C-terminus of CREB. N=8 independent experiments. **(D)** Recombinant His-SUMO-CREB directly binds to GST-LMO4 in pull-down experiments. **(E)** The interaction between CREB with LMO4 is mediated by the C-terminus of CREB (166-341 aa), but not by its N-terminus (1-165 aa). Pull-down experiments between recombinant His-SUMO-1-165-CREB, His-SUMO-166-341-CREB, and GST-LMO4. **(F)** Schematic representation of CREB and LMO4 domain structure and fusion constructs used for the experiments. Light gray boxes represent the interaction interface. **(G)** Myc-LMO4 overexpression increases CREB dependent luciferase expression in HEK293T cells expressing luciferase under the CRE promoter. Relative luciferase units in cells overexpressing Myc-LMO4 as compared to Myc-transfected controls. N=8, from two independent experiments. **(H-K)** Knockdown of LMO4 reduces nuclear pCREB immunoreactivity. **(H, J)** Representative confocal images of hippocampal neurons transfected with LMO4 shRNA construct or scrambled control (both expressing GFP under CMV promoter as a transfection control). Scale bar: 10µm. Dot plots representing the mean of nuclear **(I)** pCREB or **(K)** CREB staining intensity normalized to scrambled control. N=30-37 nuclei analyzed from at least 3 independent cell cultures. **(L, M)** SRET saturation experiments reveal that Jacob-GFP forms a triple complex with CREB-RLuc and LMO4-tagRFP. The caldendrin (CDD-GFP) was used as negative control. N=8 independent experiments. **(M)** Schematic representation of constructs used in SRET experiments. "BR" stands for binding region. **(N-P)** The N-terminus of Jacob displaces LMO4 from CREB. (N) GST-LMO4 coupled to beads was preincubated with His-SUMO-CREB and subsequently incubated with an increasing amount of MBP-Jacob-45-228. **(O)** Schematic depicts the timeline of the competition pull-down experiment. **(P)** N=6 independent experiments. **(H, J)** Lookup table indicates the pixel intensities from 0 to 255. **p<0.01, ***p<0.001, ****p<0.0001 by **(G, I, K)** two-tailed Student t-test or **(P)** one-sample t-test or **(A)** one-way ANOVA followed by Bonferroni's multiple comparisons test. All data are represented as mean ± SEM.

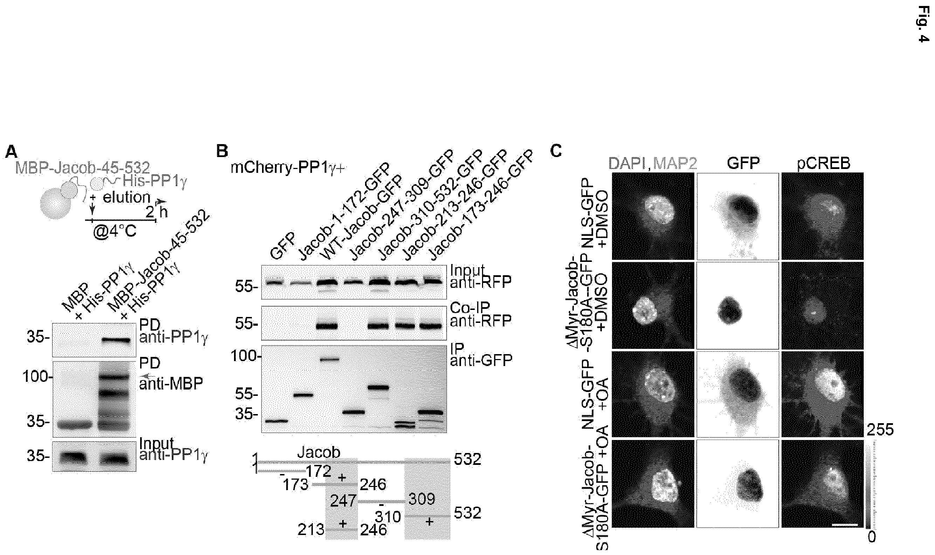

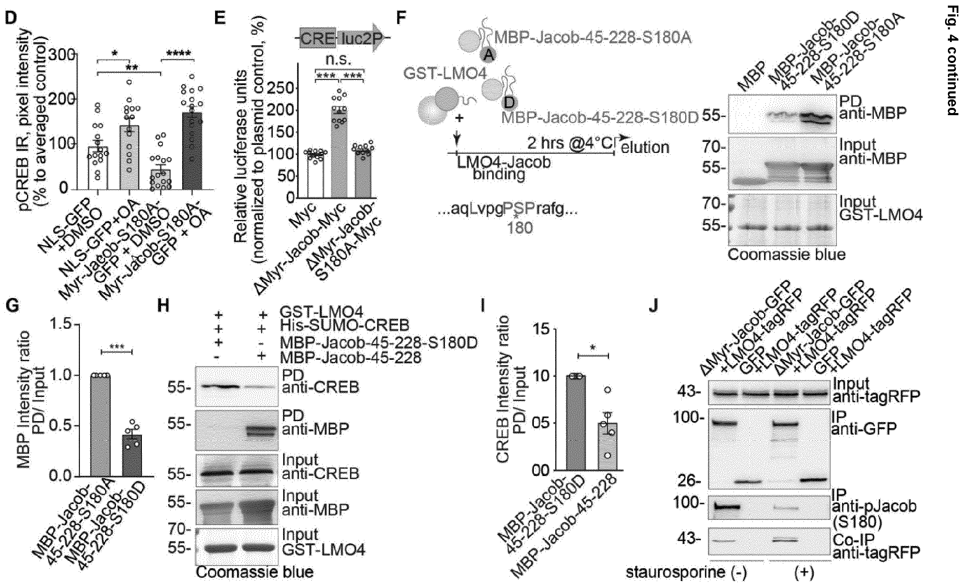

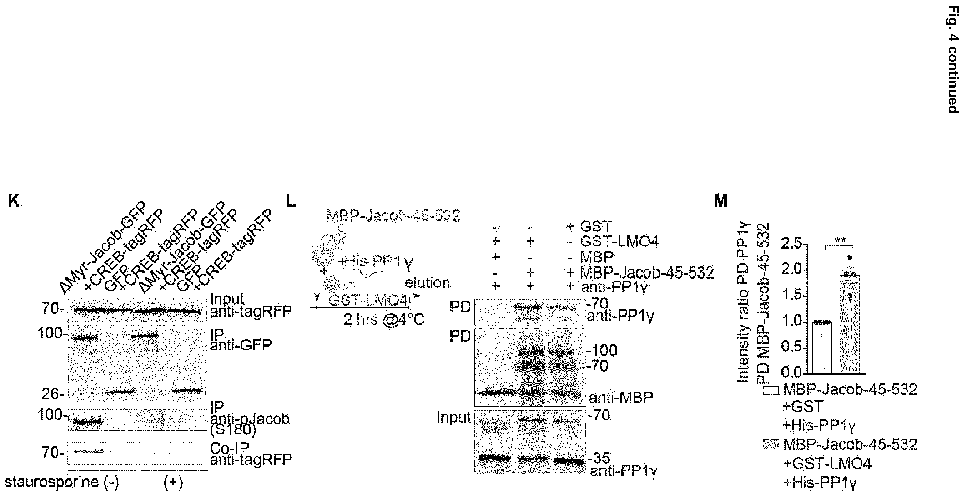

**Figure 4****:** The figure illustrates the molecular functional basis of the use of nitarsone according to embodiments of the invention. The Figure illustrates the results of the experiments described in the Examples herein, wherein the inventors found out that CREB shutoff essentially requires Jacob binding to PP1. **(A)** Pull-down experiments confirmed a direct interaction between bacterially produced recombinant MBP-Jacob-45-532 and His-PP1γ. **(B)** Mapping of the mCherry-PP1γ interaction region within the Jacob sequence revealed binding of a C-terminal fragment (Jacob-310-250-GFP) as well as the N-terminal part (Jacob-173-246-GFP) where the region between 213-246 aa is sufficient for immunoprecipitation. The gGreen boxes in schematic indicate interaction regions. **(C, D)** Treatment of hippocampal primary neurons expressing phosphodeficient mutant Jacob in the nucleus with okadaic acid rescues Jacob-induced CREB shutoff. Confocal images of pCREB immunostaining in DIV15 neurons overexpressing ΔMyr-Jacob-S180A-GFP with and without OA treatment. Scale bar: 20 µm. Lookup table indicates the pixel intensities from 0 to 255. N=14-17 nuclei analyzed from 2 independent cell cultures. **(E)** Overexpression of ΔMyr-Jacob-Myc but not the phospho-deficient mutant (ΔMyr-Jacob-S180A-Myc) positively regulates CREB dependent expression of luciferase. N=3 independent experiments. **(F, G)** Phosphodeficient N-terminus of Jacob (MBP-Jacob-45-228-180A) interacts with LMO4 stronger than its phosphomimetic form (MBP-Jacob-45-228-180D). **(G)** Quantification of MBP immunoreactivity normalized to input. N=5 independent experiments. **(H, I)** Phosphomimetic Jacob mutant (MBP-45-228-180D) does not displace LMO4 from CREB. Recombinant GST-LMO4 was coupled to beads, preincubated with His-SUMO-CREB and subsequently incubated in 1:8 ratio with MBP-Jacob-45-228 or MBP-45-228-180D. **(I)** Quantification of the CREB band intensity normalized to the input. N=5 independent experiments. **(J)** Treatment with staurosporine decreases Jacob phosphorylation level (S180) but increases its association with LMO4-tagRFP. Immunoblot of HEK293T cells extracts transfected with LMO4-tagRFP and ΔMyr-Jacob-GFP or GFP alone. **(K)** Treatment with staurosporine decreases the association of Jacob with CREB. Immunoblot of HEK293T cells extract transfected with CREB-tagRFP and ΔMyr-Jacob-GFP or GFP as a control. **(L, M)** The association of Jacob with LMO4 enhances its interaction with PP1γ in pull-down assays. **(L)** PP1γ interacts with Jacob as a dimer (70 kDa) that forms during purification. **(M)** Bar graph represents quantification of PP1γ immunoreactivity normalized to MBP-Jacob. *p<0.05, **p<0.01, ***p<0.001, ****p<0.0001 by **(G, I, M)** one-sample t-test or **(E)** one-way ANOVA followed by Bonferroni's multiple comparisons test or **(D)** two-way ANOVA followed by Bonferroni's multiple comparisons test. All data are represented as mean ± SEM.

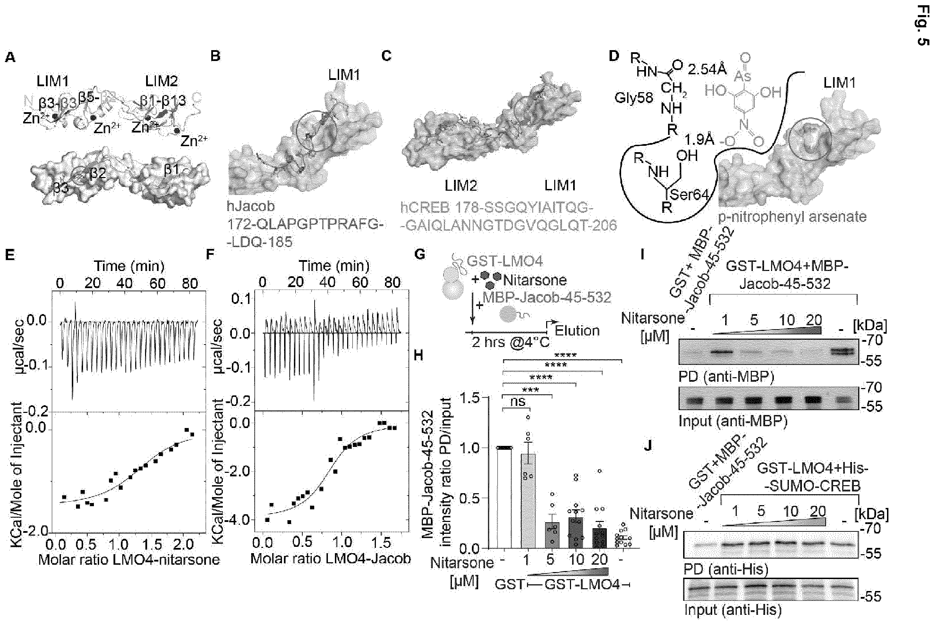

**Figure 5****:** The figure illustrates the molecular functional basis of the use of nitarsone according to embodiments of the invention. The Figure illustrates the results of the experiments described in the Examples herein, wherein the inventors found out that nitarsone disrupts LIM1-Jacob binding and rescues Aβ₁₋₄₂-induced CREB shutoff, synaptic AMPAR loss, and mEPSC amplitude impairment. **(A)** Template structure of an LMO4:peptide complex based on fusion protein LMO4:Ldb1 LID (protein databank (PDB)ID: 1RUT). LIM1-LIM2 tandem domains are folded and stabilized by 4 zinc atoms (black spheres) and bind to a 29 residues long peptide in anti-parallel orientation. The binding occurs mainly via 3 well defined β-strands (β1, β3, β4) interacting with corresponding β-strands of LMO4 (β13, β5, β3). A positional alanine scan highlighted two hydrophobic binding pockets (circles, lower panel) as hot spots of that complex allowing only residues Ile, Leu, Met, or Val to be buried in each of the pockets. The peptides are shown according to the positional alanine scan (ΔΔG (kcal/mol)) from 0% (no side chain effect) to 100% (critical conserved residue). **(B)** hJacob residues 172-185 bind to LIM1 domain (SEQ ID NO 4). **(C)** hCreb binds to LIM1 and LIM2 similar to Ldb1. **(D)** Nitarsone (p-nitrophenyl arsonic acid) fits to the hydrophobic binding pocket of LIM1 and can form two hydrogen bonds (SEQ ID NO 5: hCREB partial sequence 178-206). **(E, F)** ITC analysis of **(E)** LMO4-nitarsone or **(F)** Jacob interaction. ITC thermograms for sequential dilutions. Upper panel presents raw data, with heat pulses illustrating exothermic binding. Lower panel depicts binding curve of integrated heat measurements with the best fit using standard single-site binding model. **(G-I)** Nitarsone disrupts binding of Jacob to LMO4 in concentration-dependent manner. **(G)** Bacterially expressed GST-LMO4 was immobilized on beads and was pre-incubated with increasing concentrations of Nitarsone, and subsequently with MBP-Jacob-45-532. **(H)** MBP immunoreactivity normalized to input. N=6. **(I)** Representative immunoblot probed with anti-MBP antibody of input and pull-down with GST as a control. **(J-L)** Nitarsone does not disrupt binding of LMO4 to CREB. **(J)** Representative immunoblot probed with anti-His of input and pull-down with GST as a control. **(K)** Bacterially expressed GST-LMO4 was immobilized on beads and was pre-incubated with growing concentrations of nitarsone, and subsequently with His-Sumo-CREB. **(L)** His immunoreactivity normalized to input. N=4. **(M, N)** Nitarsone co-application prevents Aβ-induced CREB shutoff. DIV16 hippocampal cultures were treated with 5 µM Nitarsone, 500 nM Aβ₁₋₄₂, 5 µM Nitarsone with 500 nM Aβ₁₋₄₂ or vehicle control for 48h and stained for pCREB, MAP2, and DAPI. **(M)** Nuclear pCREB immunoreactivity normalized to control. N=63-67 nuclei from 3 independent cultures. **(N)** Representative confocal images. Scale bar: 10 µm. **(O, P)** 5 µM treatment with Nitarsone rescues Aβ₁₋₄₂-induced synaptic loss. DIV16 hippocampal cultures were treated with 5 µM Nitarsone, 500 nM AP₁₋₄₂, 5 µM Nitarsone with 500 nM Aβ₁₋₄₂ or vehicle control for 48h and stained for Shank3, Synaptophysin, and MAP2. **(O)** Nnumber of synaptic puncta per 1 µm. N=33-38 dendritic segments from 4 independent cell cultures. **(P)** Representative confocal images of dendritic segments. Scale bar: 5 µm. **(R, S)** 5 µM treatment with Nitarsone rescues Aβ₁₋₄₂-induced decrease of synaptic GluR1-immunoreactivity within Shank3. DIV16 dissociated, hippocampal cultures were treated with 5 µM Nitarsone, 500 nM Aβ₁₋₄₂, 5 µM Nitarsone with 500 nM Aβ₁₋₄₂ or vehicle control for 48h and stained for Shank3, surface GluR1, and MAP2 **(R)** GluR1-immunoreactivity within Shank3 signal. N=39-61 of dendritic segments from 4 independent cell cultures. **(S)** Representative confocal images of dendritic segments. Scale bar: 5 µm. **(T-X)** Nitarsone rescues decrease in mEPSCs amplitude. **(T)** Analog traces of mEPSCs recorded in DIV16 hippocampal neurons treatd with 500 nM Aβ₁₋₄₂, 5 µM Nitarsone, 5 µM Nitarsone with 500 nM Aβ₁₋₄₂ or vehicle control for 48h. **(W, X)** Cumulative probability plots of **(W)** inter-event interval or **(X)** amplitude. Quantification **of (U)** amplitude and **(V)** inter-event-interval. N=24-28 neurons from 4 independent cell cultures. **(N, S)** Lookup tab-le indicates the pixel intensities from 0 to 255. **p<0.01, ***p<0.001, ****p<0.0001 by **(H, L)** one-sample t-test or **(M, O, R, U, V)** two-way ANOVA followed by Tukey's multiple comparisons test. All data are represented as mean ± SEM.

**Figure 6****:** The figure illustrates the molecular functional basis of the use of nitarsone according to embodiments of the invention. The Figure illustrates the results of the experiments described in the Examples herein, wherein the inventors found out that treatment with Nitarsone rescues AD-related phenotype in TBA2.1 and 5xFAD mice. **(A, B) (A)** Nitarsone rescues the reduction of pCREB immunoreactivity in NeuN positive cells in CA1 of TBA2.1 mice. Cumulative frequency distribution of pCREB nuclear staining intensity. N=940-2639 nuclei from 3-9 animals. **(B)** Representative confocal images of CA1 cryosections from 11 weeks old mice stained for NeuN, DAPI, and pCREB. Scale bar: 10 µm. **(C, D) (C)** Nitarsone rescues the reduction of CREB immunoreactivity in NeuN positive cells in CA1 of TBA2.1 mice. Cumulative frequency distribution of CREB nuclear staining intensity. N=936-2349 nuclei from 3-9 animals. **(D)** Representative confocal images of CA1 cryosections from 11 weeks old mice stained for NeuN, DAPI, and CREB. Scale bar: 10 µm. **(E, F) (E)** Representative confocal images of CA1 cryosections from 18 weeks old mice stained for NeuN, DAPI, and pCREB. Scale bar: 10 µm. **(F)** Nitarsone rescues the reduction of pCREB immunoreactivity in NeuN positive cells in CA1 of 5xFAD mice. Cumulative frequency distribution of pCREB nuclear staining intensity. N=940-2639 nuclei from 5-6 animals. **(G, H) (G)** Representative confocal images of CA1 cryosections from 18 weeks old mice stained for NeuN, DAPI, and CREB. Scale bar: 10 µm. **(H)** Nitarsone rescues the reduction of CREB immunoreactivity in NeuN positive cells in CA1 of 5xFAD mice. Cumulative frequency distribution of CREB nuclear staining intensity. N=936-2349 nuclei from 5-6 animals. **(I)** Nitarsone partially rescues neuronal loss in TBA2.1 animals. The average number of NeuN-positive cells normalized to WT treated with vehicle. N=11-31 CA1 images analyzed from 3-9 animals per genotype. **(J, K) (J)** Nitarsone rescues synaptic density in SLM of CA1 of TBA2.1 mice. Number of synaptic puncta per ROI. N=9-33 ROIs from 3-9 animals **(K)** Representative confocal images of SLM from 11 weeks old mice stained for MAP2, Shank3, and Synaptophysin. Scale bar: 5 µm. **(L, M) (L)** Representative confocal images of SLM from 18 weeks old mice stained for MAP2, Shank3, and Synaptophysin. Scale bar: 5 µm. **(M)** Nitarsone rescues synaptic density in SLM of CA1 of 5xFAD mice. Number of synaptic puncta per ROI. N=12-21 ROIs from 5-6 animals. **(N, O) (N)** Nitarsone rescues late CA1-LTP impairment in TBA2.1 mice. Insets show representative fEPSPs analog traces at indicated time points: 1 = baseline, 2 = late LTP. **(O)** Averaged fEPSP slopes recorded during the last 30 min. N=14-18 slices from 5-6 mice. **(P, Q) (P)** Nitarsone rescues late CA1-LTP impairment in 5xFAD mice. Insets show representative fEPSPs analog traces at indicated time points: 1 = baseline, 2 = late LTP. **(Q)** Averaged fEPSP slopes recorded during the last 30 min. N=17-18 slices from 6 mice. **(R, S)** Nitarsone rescues short-term memory impairment in Y-maze object recognition task in **(R)** TBA2.1 N=9-14 and **(S)** 5xFAD mice. N=9-11 **(T, U)** Nitarsone rescues discrimination impairment in novel location recognition task in **(T)** TBA2.1 N=11-15 and **(U)** 5xFAD mice. N=12-13 **(V, W)** Nitarsone rescues discrimination impairment in novel object recognition task in **(V)** TBA2.1 N=11-15 and **(W)** 5xFAD mice. N=12-13 *p<0.05, **p<0.01, ***p<0.001, ****p<0.0001 by two-way ANOVA followed by Bonferroni's multiple comparisons test. All data are represented as mean ± SEM.

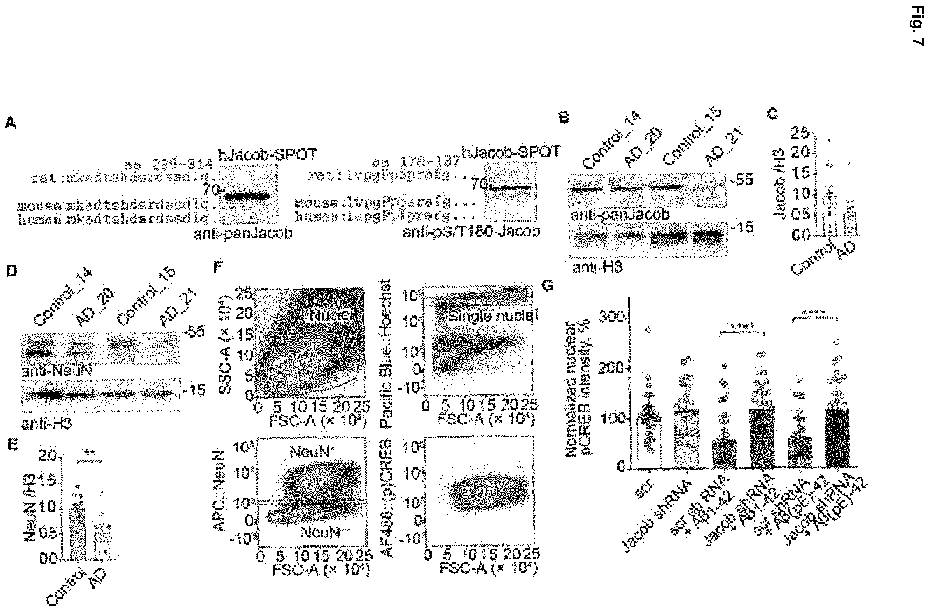

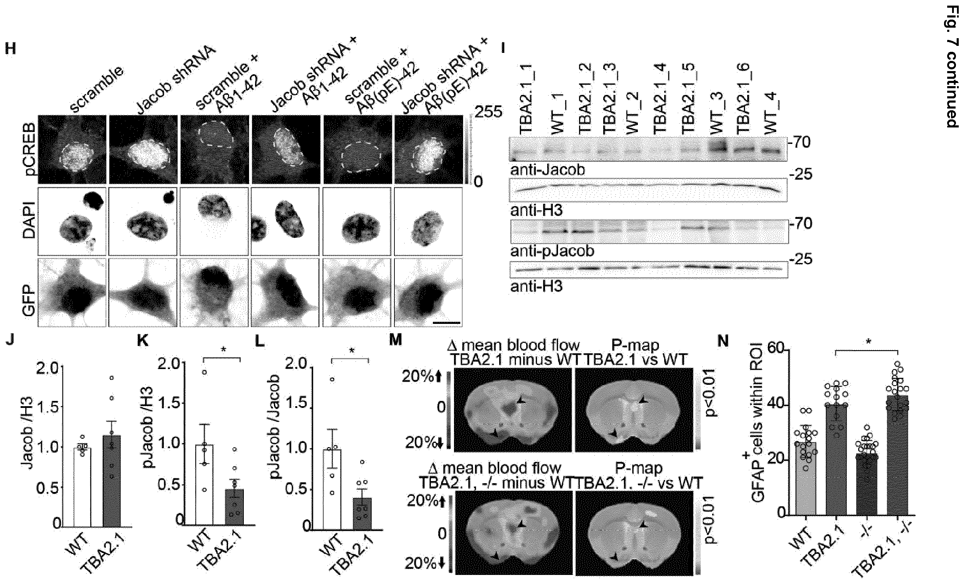

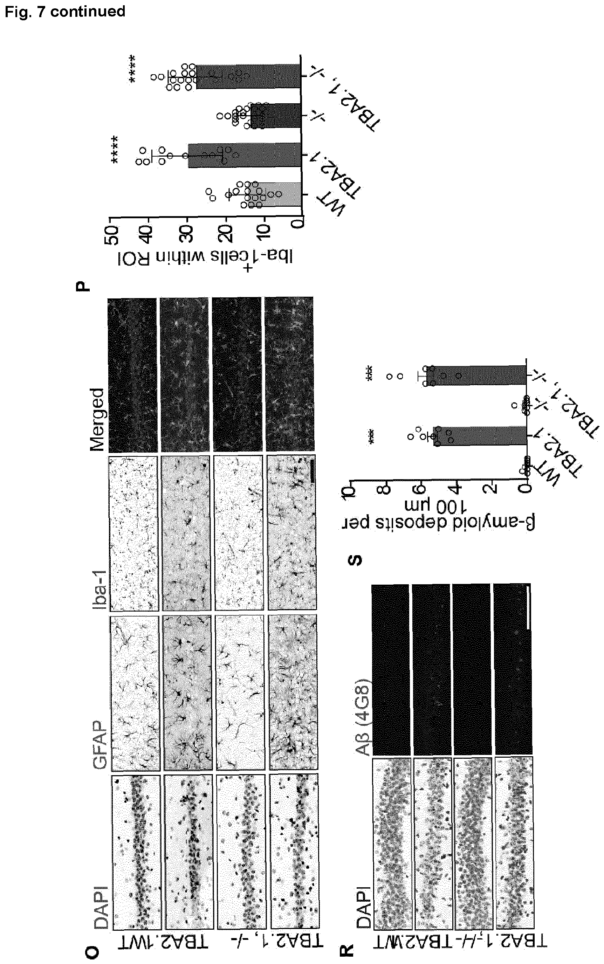

**Figure 7****:** The figure illustrates the molecular functional basis of the use of nitarsone according to embodiments of the invention and provides supplemental information to Fig. 1. The Figure illustrates the results of the experiments described in the Examples herein, wherein the inventors found out that Jacob-regulated CREB phosphorylation decreases in an animal model of Alzheimer's disease and in Alzheimer's disease patients. **(A)** Schematic represents the immunoprecipitation approach employing Spot-nano Trap coupled to magnetic agarose used for the enrichment of hJacob expressed in HEK293T cells for antibody detection. Rat amino acid sequence of Jacob used for generation of pan-Jacob antibodies is conserved throughout the species and the antibody effectively detects human Jacob. Despite variability within the peptide amino acid sequence used for the production of polyclonal pJacob antibodies they do recognize the human protein. **(B, C)** Total Jacob protein levels are not significantly reduced in brain samples from Alzheimer's disease patients as compared to the control group. **(C)** Bar plots representing the quantification of WB immunodensity for particular protein normalized to H3 and control patients. N=11-12 protein extracts from different subjects. **(D, E)** Total NeuN protein levels are significantly reduced in brain samples from Alzheimer's disease patients as compared to the control group. **(C)** Bar plots representing the quantification of WB immunodensity for particular protein normalized to H3 and control patients. N=11-12 protein extracts from different subjects. **(F)** Scatter plots representing gating strategy used in FACS experiments for neuronal pCREB and CREB immunoreactivity quantification. **(G, H)** Jacob shRNA knockdown prevents Aβ-induced CREB shutoff. Representative confocal images of hippocampal neurons transfected with Jacob-shRNA construct or scrambled (scr) shRNA control (both expressing GFP) and treated with oligomeric preparations of Aβ₁₋₄₂ or Aβ_{3(pE)-42}. **(H)** Contrary to the scr shRNA, neurons transfected with Jacob knockdown construct did not display reduction of pCREB staining intensity after treatment with Aβ₁₋₄₂ or Aβ_{3(pE)-42}. Bar plot of mean nuclear pCREB intensity normalized to scramble untreated control. Scale bar: 10 µm. N=29-39 nuclei from 2 independent experiments. Lookup table indicates the pixel intensities from 0 to 255. **(I-L) (I)** pJacob level and pJacob/panJacob ratio are decreased in TBA2.1 mouse line compared to WT animals. Representative images of the immunoblot probed with antibodies against pJacob, pan-Jacob, and re-probed with Histone3 (H3). The protein quantification was normalized to H3. **(J-L)** Bar plots representing the quantification of WB immunodensity of **(J)** Jacob level, **(K)** pJacob level and **(L)** pJacob/Jacob ratio normalized to H3. N=5-7 hippocampal, protein extracts. **(M)** Significant changes in cerebral blood flow between TBA2.1 and WT, TBA2.1, -/- and WT as determined by SPECT measurements. The statistically significant differences between TBA2.1 or double transgenic animal and WT were detected in lateral septal nucleus and the diagonal band nucleus. (p<0,01) by two-tailed Student t-test. **(N)** Bar plot representing the number of GFAP positive cells per rectangular region of interest. N=17-24 cryosections from 5-7 animals per genotype. **(O)** Representative confocal images of distal CA1 sections from 13 weeks old mice stained for GFAP, DAPI and Iba-1. Scale bar: 100 µm. **(P)** Bar plot representing the number of Iba-1 positive cells per rectangular region of interest. N=17-26 cryosections from 5-7 animals per genotype. **(R)** Confocal images averaged from two sections of the molecular layer of 13 weeks old mice distal CA1 labelled for amyloid-β (4G8 antibody) and co-stained with DAPI. Scale bar: 100 µm. **(S)** Bar plot representing the number of amyloid-β positive puncta per 100 µm. N=8, number of cryosections from 2 animals per genotype. *p<0,05, **p<0.01, ***p<0.001, ****p<0.0001 by **(J, K, L)** two-tailed Student t-test or **(N, P, S)** two-way ANOVA followed by Bonferroni's multiple comparisons test. All data are represented as mean ± SEM.

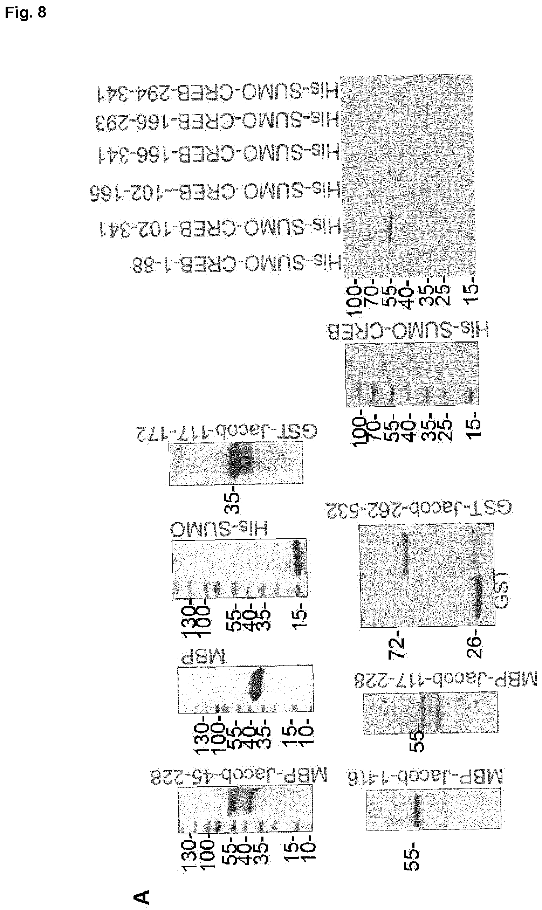

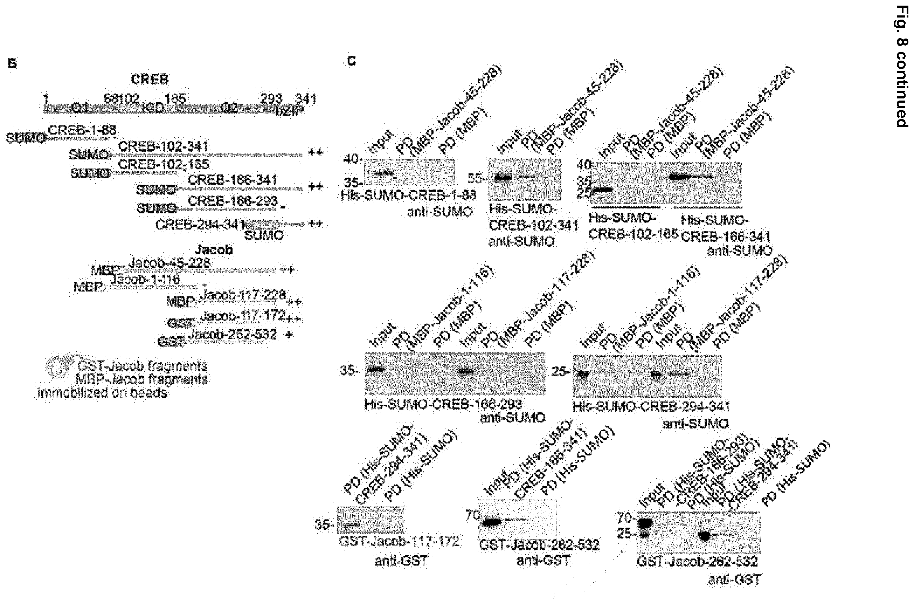

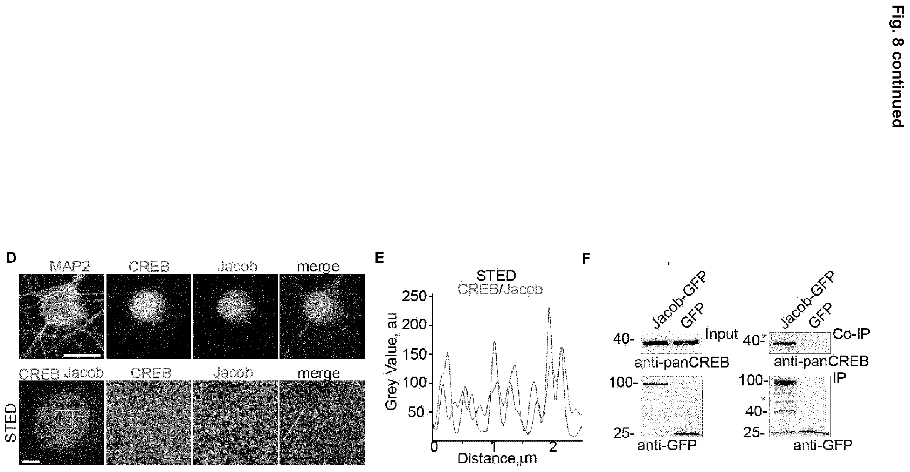

**Figure 8****:** The figure illustrates the molecular functional basis of the use of nitarsone according to embodiments of the invention and provides supplemental information to Fig. 2. The Figure illustrates the results of the experiments described in the Examples herein, wherein the inventors could confirm Jacob-CREB interaction, mapping of the binding interfaces between Jacob and CREB. **(A)** Coomassie blue staining depicting the purity of bacterially produced proteins used for pull-down assays between CREB and Jacob. **(B)** Scheme representing the constructs used for mapping of the interaction sites in Fig. 1D and 7B. **(C)** The N-terminus of Jacob (117-172 aa) interacts with the bZIP domain of CREB, but not with the Q1 (1-88 aa), KID (102-165 aa), or Q2 (166-293 aa) domains. The C-terminus of Jacob (262-532 aa) shows weaker binding to the bZIP domain of CREB. Images of immunoblots representing pull-down assays performed with Jacob and CREB protein fragments depicted in the panel B. **(D, E)** Confocal and STED images show an association of CREB with Jacob in the nucleus of DIV16 hippocampal primary neurons. **(D)** The upper panel represents deconvolved confocal images. Lower panels depict deconvolved STED images. Scale bars: 20 µm and 5 µm respectively. Inserts are denoted by a white square. **(E)** Line profiles indicate the overlap of relative intensities for CREB and Jacob along a 2,5 µm line. **(F)** Endogenous CREB co-immunoprecipitates with overexpressed Jacob-GFP, but not GFP from HEK293T cell extracts. The asterisk denotes the CREB band from a membrane subsequently re-probed with an anti-GFP antibody.

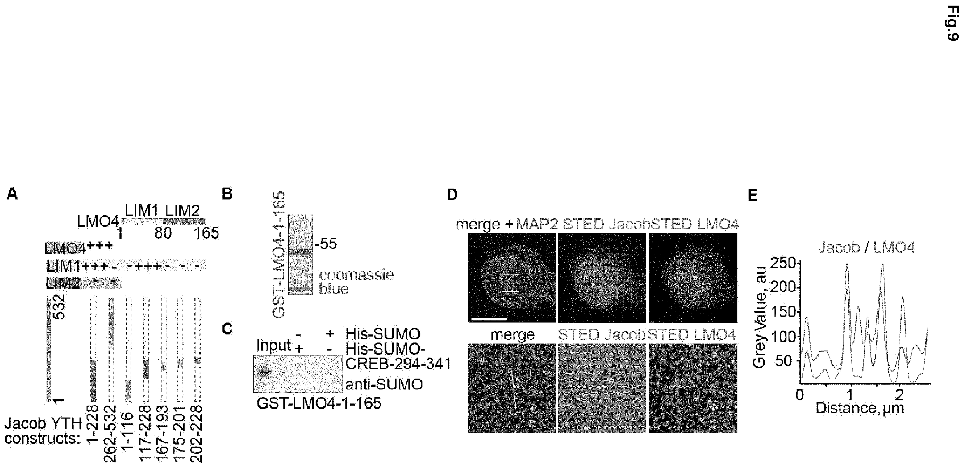

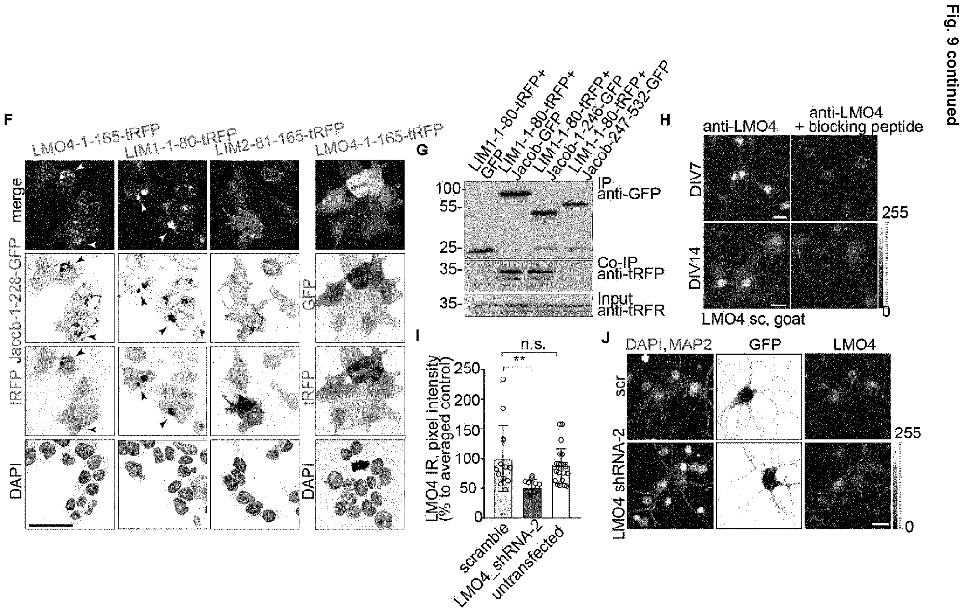

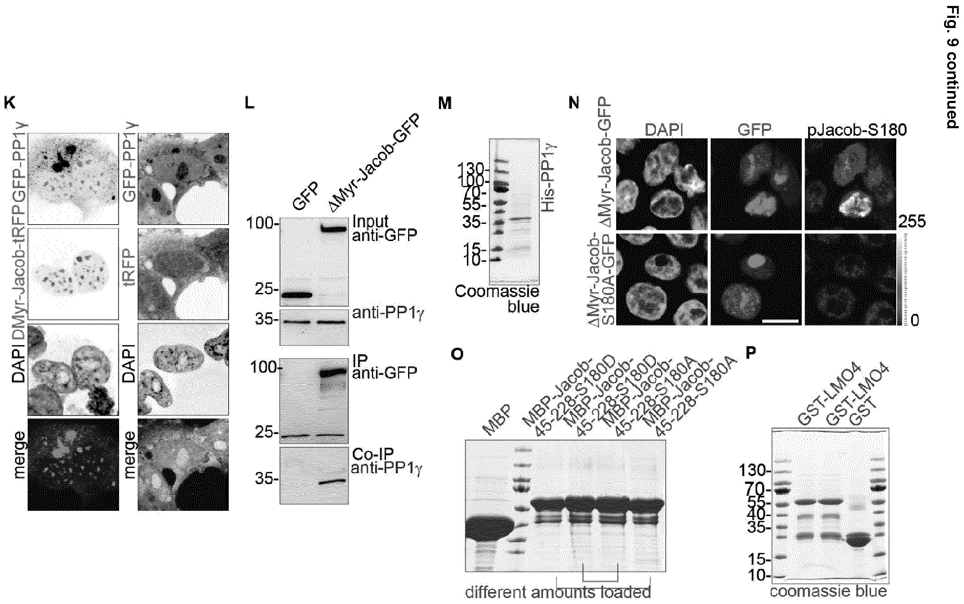

**Figure 9****:** The figure illustrates the molecular functional basis of the use of nitarsone according to embodiments of the invention and provides supplemental information to Figs. 2-4. The Figure illustrates the results of the experiments described in the Examples herein, wherein the inventors could confirm LMO4-Jacob interaction, LMO4 antibody specificity and quantification of LMO4 knockdown efficiency, confirmation of Jacob-PP1 interaction, protein purity and loading controls for pull down experiments and studies on the interaction and network activity depending on Jacob S180 phosphorylation. **(A)** Jacob-117-228 interacts with the LIM1 domain of LMO4 in Y2H. (+++) indicates a strong interaction while (-) indicates no interaction. Evaluation was based on the number of colonies growing in triple drop-out media. **(B)** Coomassie blue stained gel showing purity of GST-LMO4 used in the pull-down experiments. **(C)** Pull-down experiments revealed no interaction between His-SUMO-CREB-294-341 with GST-LMO4. **(D, E)** Super-resolution STED imaging revealed association of Jacob with LMO4 in the nucleus **(D).** DIV16 primary hippocampal neurons stained with antibodies against MAP2, LMO4, pan-Jacob. Upper panel - scale bar:10 µm, lower panel (inserts 5 µm x5 µm - denoted by the white square). **(E)** Line profiles indicate relative intensities for deconvolved STED channels along a 2.5 µm line. **(F)** Jacob-1-228-GFP co-recruits the LIM1 (1-80), but not LIM2 (81-165) domain of LMO4. Confocal images of HEK293T cells co-transfected either with Jacob-1-228-GFP or GFP together with LMO4 constructs. Arrows indicate co-recruitment. Scale bar: 40 µm. **(G)** LIM1-1-80-tRFP co-immunoprecipitates with Jacob-1-246-GFP, but not Jacob-247-532-GFP from HEK293T cell extracts. **(H)** A goat C-15 LMO4 antibody (Santa Cruz) was used to stain LMO4 in hippocampal neurons (DIV 7 and DIV 14) in the presence or absence of the blocking peptide. Scale bars: 15 µm. **(I)** Representative, confocal images of hippocampal neurons transfected with shRNA targeting LMO4 or scrambled control. Reduction of nuclear LMO4 level was confirmed in immunocytochemistry with the goat anti-LMO4 (sc C-15). Scale bar:10 µm. **(J)** Nuclear LMO4 staining intensity was downregulated in neurons expressing shRNA targeting LMO4 mRNA compared to scrambled-transfected (scr shRNA) or non-transfected cells. Data represented as mean ± SEM. n=12-23 nuclei. **p<0,021 by Kruskal-Wallis test followed by Dunn's multiple comparison test. **(K)** Confocal images of HEK293T cells overexpressing GFP-PP1γ together with ether ΔMyr-Jacob-tagRFP or tRFP control revealed nuclear co-clustering of both proteins. Scale bar: 20 µm. **(L)** ΔMyr-Jacob-GFP but not GFP co-immunoprecipitate with endogenous PP1γ from HEK293T cells extracts. **(M)** Coomassie blue stained gel showing purity of commercially available His-PP1γ. **(N)** Nuclear Jacob (ΔMyr-Jacob-GFP), but not nuclear phosphodeficient mutant (ΔMyr-Jacob-S180A-GFP) expressed in HEK293T cells is phosphorylated. Confocal images of HEK293T cells overexpressing Jacob and immunostained with anti-pS180Jacob antibodies. Scale bar: 10 µm. **(O)** Image of gels stained with coomassie blue showing the purity of bacterially produced Jacob mutants used for pull-down assay. **(P)** Images of gels stained with coomassie blue showing inputs for bacterially produced GST-LMO4 coupled to beads used for pull-down assay with Jacob and PP1γ.

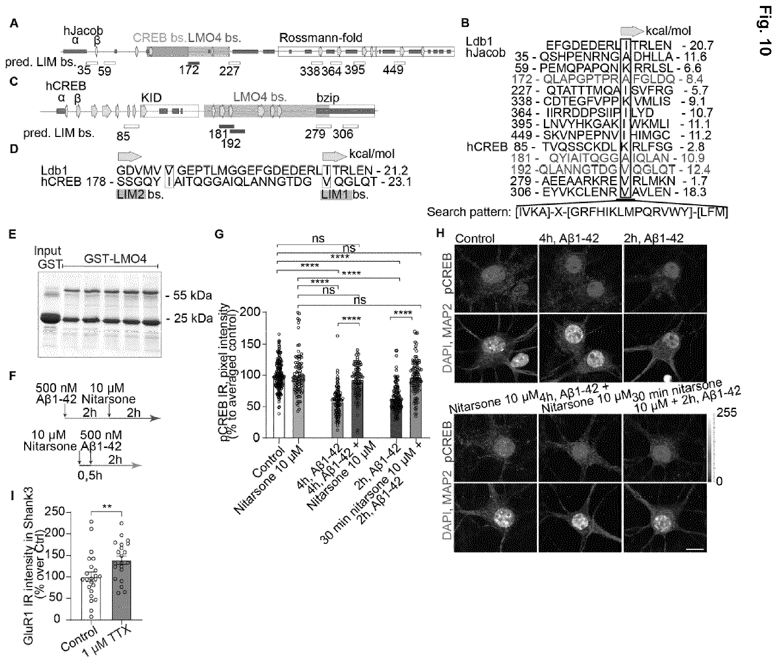

**Figure 10****:** The figure illustrates the molecular functional basis of the use of nitarsone according to embodiments of the invention and provides supplemental information to Fig. 5. The Figure illustrates the results of the experiments described in the Examples herein, wherein the inventors could show that Nitarsone disrupts Jacob-LMO4 interaction and rescues acute CREB shutoff. **(A-D)** Predicted binding sites for LMO4 LIM domains in Jacob and CREB. **(A)** Schematic structure of human Jacob showing predicted secondary structures (helices, dark greay; β-strands, arrows) and experimentally determined binding regions for CREB and LMO4. The C-terminus of Jacob is predicted to have a Rossmann-fold similar to caspases. **(B)** The LIM1 binding peptide of Ldb1 (SEQ ID NO 6) is aligned to 8 sequences of Jacob that match the search pattern for the conserved hydrophobic residues and the adjacent β-strand (SEQ ID NO 7 to 14, partial sequences of hJacob starting from amino acid numbers 35, 59, 172, 227, 338, 364, 395 and 449, respectively). Structures of LIM1 :peptides were modelled and free energy ΔΔG were calculated. Only the peptide starting at 172 lies within the LMO4 binding region. In human CREB 5 matching peptides were identified (SEQ ID NO 15 to 19; partial sequences of hCREB starting from amino acid numbers 85, 181, 192, 279 and 306, respectively). **(C)** Schematic structure of human CREB with labeled LMO4 binding region and known KID and bZIP domains. **(D)** The two peptides starting at 181 and 192 are within the LMO4 binding region and align to Ldb1 peptide (SEQ ID NO 20) where 181 binds to LIM2 and 192 to LIM1 (SEQ ID NO 21; partial sequence of hCREB starting from amino acid number 178). **(E)** Image of gels stained with coomassie blue showing the purity of bacterially produced GSt-LMO4 used for pull-down assay. **(F-H)** Acute treatment with 10 µM Nitarsone rescuses 500 nM Aβ₁₋₄₂-induced CREB shutoff. **(F)** Scheme of the experimental design. The dissociated, hippocampal cell cultures at DIV16 were either pre-treated for 30 min with 10 µM Nitarsone and subsequently 2h with 500 nM Aβ₁₋₄₂ or the drug was added 2h post the 500 nM Aβ₁₋₄₂ treatment. The pCREB immunoreactivity was measured in comparison to Vehicle control. **(G)** Bar pot representing nuclear pCREB immunostaining intensity normalized to vehicle control. N=88-101 from 5-7 independent cell cultures. ****p<0,0001 by two-way ANOVA with Sidak's post hoc test. **(H)** Representative confocal images of hippocampal. Lookup table indicates the pixel intensities from 0 to 255. Scale bar: 10µm. **(I)** Treatment with 1 µM TTX induced upregulation of GluR1 surface expression. N=21-23 dendritic segments from 3 independent cell cultures. **p<0,01 by two-tailed Student t-test. All data are represented as mean ± SEM.

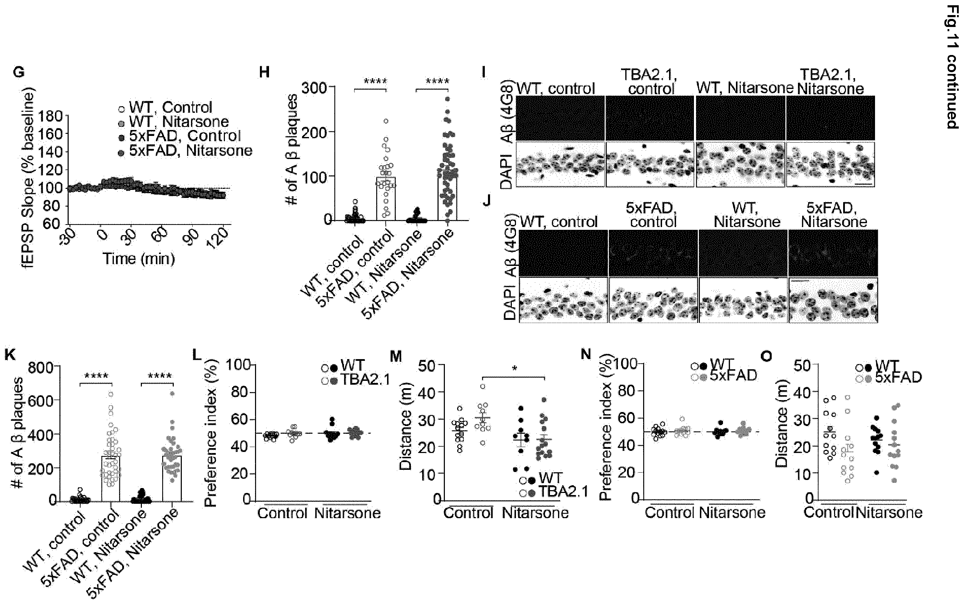

**Figure 11****:** The figure illustrates the molecular functional basis of the use of nitarsone according to embodiments of the invention and provides supplemental information to Fig. 5. The Figure illustrates the results of the experiments described in the Examples herein, wherein the inventors could show that treatment with Nitarsone rescues AD-related phenotype in TBA2.1 and 5xFAD mice. **(A)** Scheme representing the timeline of treatment with Nitarsone of TBA2.1 and 5xFAD mice. **(B, C)** TBA2.1 mice do not display neuronal loss at the beginning of the Nitarsone treatment. **(B)** Bar graph representing the average number of NeuN-positive cells normalized to WT treated with vehicle. N=2-4 mice **(C)** Representative confocal images of distal CA1 cryosections from 4 weeks old mice stained for NeuN, DAPI, and CREB. Scale bar: 50 µm. **(D, E)** 5xFAD mice do not display neuronal loss at the end of the Nitarsone treatment. **(D)** Bar graph representing the average number of NeuN-positive cells normalized to WT treated with vehicle N= 2 mice. **(E)** Representative confocal images of distal CA1 cryosections from 19 weeks old mice stained for NeuN, DAPI, and CREB. Scale bar: 50 µm. **(F, G)** Basal synaptic transmission is not affected by bath application of Nitarsone in **(F)** TBA2.1 and **(G)** 5xFAD mice. TBA2.1: N=14-18 slices from 5-6 mice and 5xFAD: N=17-18 slices from 6 mice. (H-**K)** Nitarsone treatment does not change amyloid load in **(H, I)** TBA2.1 and **(J, K)** 5xFAD mice. **(H, K)** Bar plot representing the number of amyloid-β positive puncta. **(H)** TBA2.1 N=18-50 CA1 regions 3-9 animals per genotype and **(K)** 5xFAD N=29-39 CA1 regions 5-6 animals per genotype. **(I, J)** Confocal images averaged from two sections of the molecular layer of CA1 labelled for amyloid-β (4G8 antibody) and co-stained with DAPI. Scale bar: 100 µm. **(L, M) (L)** Nitarsone treatment does not influence preference index and **(M)** slightly normalizes increased distance travelled during open field arena exploration by TBA2.1 mice. **(N, O) (N)** Nitarsone treatment does not influence preference index and **(O)** slightly normalizes increased distance travelled during open field arena exploration by TBA2.1 mice. *p<0.05, ****p<0.0001 by two-way ANOVA followed by Bonferroni's multiple comparisons test. All data are represented as mean ± SEM.

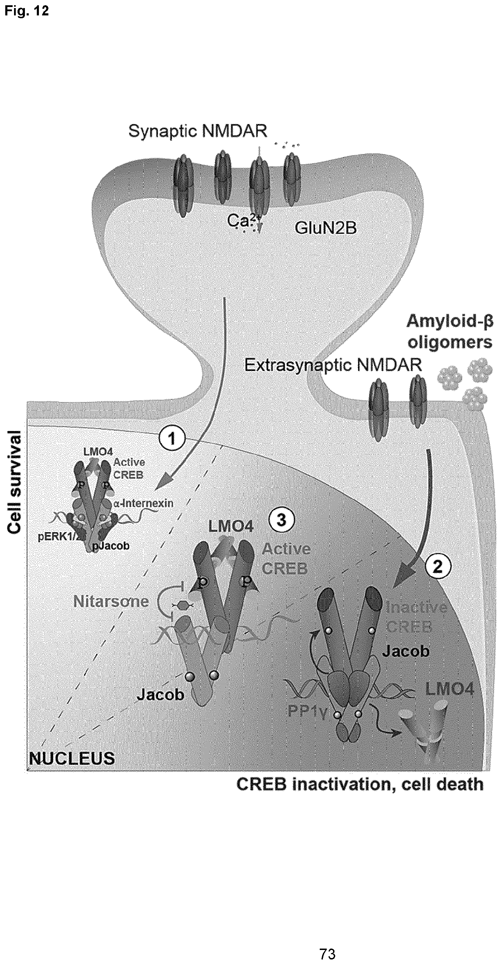

**Figure 12****:** Graphic representation of scientific principle behind embodiments of the invention. (1) NMDAr-dependent synaptic signals lead to Jacob-dependent import of kinase, CREB activation, and subsequent expression of pro-survival genes. (2) Amyloid-β oligomers lead to NMDAR-dependent transport of Jacob protein complex including phosphatase (PP1). In addition, Jacob displaces LMO4 from CREB, leading to its inactivation, and, ultimately, cell death. (3) Treatment with nitarsone inhibits Jacob-dependent displacement of LMO4 from CREB, preserving its activation.

### EXAMPLES

The invention is further described by the following examples. These are not intended to limit the scope of the invention but represent preferred embodiments of aspects of the invention provided for greater illustration of the invention described herein.

### Methods employed in the Examples

**Table 1. Summary of cases used in the Examples.**

| Number | Gender | Age | Group |
|---|---|---|---|
| 1 | m | 93 | Alzheimer's disease |
| 2 | w | 74 | Control |
| 3 | w | 86 | Alzheimer's disease |
| 4 | m | 67 | Control |
| 6 | m | 50 | Control |
| 7 | m | 52 | Control |
| 8 | m | 76 | Alzheimer's disease |
| 9 | m | 65 | Control |
| 10 | w | 83 | Alzheimer's disease |
| 11 | w | 81 | Alzheimer's disease |
| 12 | w | 77 | Alzheimer's disease |
| 13 | w | 84 | Control |
| 14 | m | 82 | Control |
| 15 | m | 44 | Control |
| 16 | w | 53 | Control |
| 17 | w | 72 | Control |
| 18 | w | 84 | Control |
| 19 | m | 69 | Alzheimer's disease |
| 20 | m | 73 | Alzheimer's disease |
| 21 | w | 64 | Alzheimer's disease |
| 22 | w | 88 | Alzheimer's disease |
| 23 | w | 82 | Alzheimer's disease |
| 24 | m | 80 | Alzheimer's disease |

### Human subjects

The temporal cortex (area 22) biospecimens (Table 1) were provided by the Brain Banking Centre Leipzig of the German Brain-Net, operated by the Paul Flechsig Institute of Brain Research (Leipzig University). The diagnosis and staging of Alzheimer's disease cases was based on the presence of neurofibrillary tangles (Braak and Braak, 1991) and neuritic plaques in the hippocampal formation and neocortical areas as outlined by the Consortium to establish a registry for Alzheimer's disease (CERAD; (Mirra et al., 1991)) and met the criteria of the National Institute on Aging on the likelihood of dementia (The National Institute on Aging, 1997). The exact sex, age, post mortem delay of sample collection can be found in Table 1.

### Animals

Animals were maintained in the animal facility of the Leibniz Institute for Neurobiology, Magdeburg. Jacob/Nsmf knockout (homozygous labelled as -/-) animals were characterized previously (Spilker et al., 2016) and TBA2.1 (homozygous labelled as TBA2.1) mice (Alexandru et al., 2011). To generate double transgenic animals (animals homozygous for both mutations labelled as TBA2.1, -/-) Jacob/Nsmf heterozygous mice were crossed with heterozygous TBA2.1 mice. The 5xFAD mice (Oakley et al., 2006) were purchased from Jackson Laboratories. All lines had a C57BL/6J background. Mice were housed under controlled environmental conditions (12 h, light-dark cycle, with lights on at 06:00 a.m.), with free access to food and water. Unless indicated otherwise, the animals were housed in groups up to 5 mice per cage. All animals were genotyped prior and after the experiment.

### Murine organotypic hippocampal slice culture (OHSC)

OHSC were prepared according to previously published (Grochowska et al., 2017). Slices were obtained from P7-P9 mice of both sexes (Jacob/Nsmf knockout or WT littermates as a control). Animals were decapitated, brains removed, and hippocampi dissected under a binocular. 350-400 µm thick Perpendicular slices were cut using a McIlwain tissue chopper (Mickle Laboratory Engineering). Slices were cultured on millicell membranes (3 slices per membrane, Merck Milipore) in 6 well-plates in 1 ml of medium 50% minimal essential medium (Gibco), 25% heat inactivated horse serum (Gibco), 25 mM glucose, 2 mM glutamine, 25 mM HEPES, 1xB27 (Gibco), penicillin/streptomycin (100 U/ml). Cultures were grown at the 37°C, 5% CO2, 95% humidity. Every 3rd day the 700 µl of the medium was exchanged.

### Primary hippocampal cultures

Hippocampal and cortical cultures were prepared from Wistar rat embryos (E18) of mixed sex as described previously (Spilker et al., 2016). Briefly, dissected were digested for 15 min with trypsin at 37°C. Neurons were plated on plastic 12-well dishes (Greiner) on glass coverslips coated with poly-L-lysine (Sigma-Aldrich) at a density of 60 000 cells/well in DMEM medium (Gibco, Thermo Fisher Scientific) supplemented with 10% FCS, 1x penicillin/streptomycin, and 2 mM glutamine. After 1h incubation (at the 37°C, 5% CO2, 95% humidity) cells were kept in BrainPhys medium supplemented with 1%SM1 (Stemmcell Technologies), 0.5 mM Glutamine (Gibco) at 37°C, 5% CO2 and 95% humidity.

### Cell lines

HEK293T cells were maintained DMEM medium (Gibco, Thermo Fisher Scientific) supplemented with 10% FCS, 1x penicillin/streptomycin, and 2 mM glutamine at the 37°C, 5% CO2, 95% humidity.

### Structural Modelling

Structures of LMO4:peptide complexes were modeled using coordinates of LMO4:Ldb1 complex (PDBId: 1RUT) using Swiss-PDB Viewer v4.1 (Guex and Peitsch, 1997; Johansson et al., 2012). Positional refinement and calculation of free binding energy ΔΔG of LMO4:peptide complexes were performed by FoldX v5 (Schymkowitz et al., 2005). Donor and acceptor atoms of LMO4 LI1:Jacob peptide complexes were identified using ZINCPharmer and ZINC15 (11/20) (Koes and Camacho, 2012). Nitarsone (4-Nitrophenylarsonic acid) has been geometrically optimized and generated in PDB format by Avogadro v1.2 (Hanwell et al., 2012) and used as ligand for LMO4 LIM1 by molecular docking program AutoDock Vina v1.1.2 (Eberhardt et al., 2021). Secondary structures of full-length hJacob and hCreb were predicted with PsiPred v1.1.2 (McGuffin et al., 2000). RaptorX (12/20) (Källberg et al., 2012) was used to predict the structure of Jacob C-terminus. Structures were visualized using Open-source PyMol v2.5 (pymol.org).

### Yeast-Two-Hybrid screening

Yeast-Two-Hybrid screening for Jacob interaction partners was performed using fusion vectors (bait vector pGBKT7 (Jacob fragments (in aa): 1-228, 262-532; LMO4), prey vector pGADT7 (Jacob fragments (in aa): 1-228, 1-116, 117-228, 167-193, 175-201, 202-228, 117-228, 167-193, 175-201, 202-228; LMO4 fragments (in aa): 1-80, 81-165) using MATCHMAKER Two-Hybrid System 3 (Takara Bio Europe/Clontech, France). Co-transformed yeasts were assayed for growth on quadruple drop-out medium (SD/-Ade/-His/-Leu/-Trp) and additionally for LacZ reporter activation according to the manufacturer's protocol.

### Recombinant protein production

GST-tagged recombinant proteins (LMO4) were produced and purified as described previously (Dieterich et al., 2008; Karpova et al., 2013). For protein production E. *coli* BL21 (DE3) strain was used. Following induction with with 0.3 mM isopropyl-beta-D-thiogalactoside (IPTG) at 18°C cells were pelleted by centrifugation at 6.000 × g for 15 min and purified from the soluble fraction by glutathione-Sepharose chromatography (elution buffer: 50 mM Tris-HCI, 10 mM reduced glutathione, pH 8.0). For MBP-tagged recombinant proteins (Jacob fragments (in aa): 45-228, 1-116, 117-228, 262-532, 262-532, 45-228, 45-228-S180D (phosphomimetic mutant), 45-228-S180A (phosphodeficient mutant)) lysis was done in 20 mM Tris buffer (pH 7.4), 200 mM NaCl, 1 mM DTT and 1 mM EDTA. Protein was eluted with lysis buffer+10 mM maltose. His-SUMO-tagged recombinant proteins (CREB, CREB fragments (in aa): 1-88, 1-165, 1-293, 89-314, 89-165, 166-341, 166-293, 294-341) were purified from native conditions in which lysis buffer, wash, and elution buffer all have 50 mM NaH2PO4 (pH 8.0) and 300 mM NaCl with various concentrations of imidazole (10, 20 and 250 mM respectively). All Protease inhibitor (Complete, Roche) was used in all mentioned buffers. MBP-tagged PP1γ was obtained from Creative BioMart. The purity of the protein was checked on SDS-PAGE gels stained and Coomassie blue staining.

### Isothermal titration calorimetry (ITC)

LMO4-Nitarsone (98%, ABCR Gute Chemie) binding affinity was measured using VP-ITC calorimeter (MicroCal) and data were analysed by MicroCal LLC ITC software (MicroCal). Both purified GST-LMO4 protein and Nitarsone were prepared in 25 mM Tris buffer (pH 7.4) containing 50 mM NaCl. 20 µM GST-LMO4 or buffer control (to calculate heat of dilution) was titrated against 180 µM Nitarsone. LMO4 and Jacob binding affinity was measured by analysing binding isotherms for titration of 5 µM Jacob and 40 µM LMO4. To analyze the influence of Nitarsone on Jacob-LMO4 interaction, LMO4 protein was saturated with Nitarsone, and subsequently the Jacob protein was injected. Typically for an ITC experiment 30 injections of 10 µl each were made at 180 sec intervals. Heat change was determined by integration of the obtained peak of differential power by the instrument. Different parameters like binding enthalpy (ΔH), dissociation constant (Kd), and stoichiometry were calculated.

### Pull-down assays

Pull-down assays performed as described previously (Dieterich et al., 2008; Karpova et al., 2013). Briefly, protein amounts ranging from 1-10 µg along with the equivalent amount of the control tag protein was bound on the respective beads and was incubated with 5% BSA for 1 h at room temperature (RT). 200 ng-10 µg of the second recombinant protein was incubated with the resin bound protein in 1ml TBS buffer for 1h either at room temperature or 3 h at 4°C. After three washing steps with TBS buffer containing 0.2-0.5 % Triton X100, the complex was eluted in 2X SDS sample buffer (250 mM Tris-HCI, pH 6.8, 1% (w/v) SDS, 40% (v/v) Glycerol 20% (v/v) β-mercaptoethanol, 0,004 % Bromophenol Blue). For competition pull-down assay 20µg of GST-LMO4 and equimolar amounts of GST control protein were bound to glutathione beads followed by 5% BSA blocking and washing with 50 mM Tris-CI, pH 7.5, 150 mM NaCl. Different combinations of recombinant proteins (i.e., HIS-SUMO-CREB; 1:1 of His-SUMO-CREB and MBP-Jacob-45-228; 1:4 of His-SUMO-CREB and MBP Jacob-45-228; and 1:8 of His-SUMO-CREB and MBP-Jacob-45-228) were incubated with GST-LMO4 and GST (control protein) immobilized on Protino Glutathione Agarose 4B beads (Macherey-Nagel). Probes were eluted with 25µl of 2× SDS sample buffer after incubation and washing steps (washing buffer: 50 mM Tris pH 7.4, 500 mM NaCl, 0.1 % Triton X100 and protease inhibitor without EDTA). For pull-down assays with Nitarsone 10-20 µg of GST-LMO4 or GST control protein were immobilized on glutathione beads followed by overnight incubation with 1-20 µM of Nitarsone solution at 4°C. Equimolar MBP-Jacob-45-228 were added to the solution and incubated rotating for 2 h and washing was performed with 20 mM Tris-CI (pH 7.4), 150 mM NaCl, 0.2 % TritonX-100 containing phosphatase and protease inhibitors. Complex was eluted using 2X SDS sample buffer.

### Heterologous co-immunoprecipitation

The constructs (LMO fragments (in aa) tagged with tRFP: 1-80 or 81- with GFP, or Jacob fragments (in aa): tagged with GFP: 45-172, 173, 246; mcherry-PP1γ (Liu et al., 2010) with GFP or Jacob or Jacob fragments (in aa) tagged with GFP: 1-172, 247-309, 310-532, 213-246, 173-246) were heterologously expressed in HEK293T cells. Cells were harvested in cold TBS buffer containing protease inhibitors (Roche) and phosphatase inhibitors (Roche), in a 1g/10ml ratio 48 h after transfection. The pelleted cells were lysed in cold RIPA buffer (50 mM Tris-HCI (pH 8.0), 150 mM NaCl, 1% NP40, 0,5% doxycycline (DOC), 0,1%, sodium dodecyl sulfate, (SDS), protease and phosphatase inhibitors, pH 7.4) for 1,5 h in 4°C, rotating. The lysates were subsequently centrifuged at 20 000 g for 20 min. The supernatant was incubated with 25 µl of µMACS Anti-GFP MicroBeads (Miltenyi Biotec) for 40 min in 4°C, rotating. Beads were collected with the use of µMACS magnetic column and washed twice with 400 µl of RIPA buffer and 300 µl of 20 mM Tris-HCI (pH 7.5).

The phospho-dependent association of overexpressed nuclear Jacob was assessed by heterologous co-immunoprecipitation from HEK293T cells pre-treated with 20 nM staurosporine for 3 h.

### Characterization of Jacob antibodies

For initial characterization of anti-panJacob and pJacob (S180) antibodies for the detection of human protein HEK293T cells were transfected with the plasmid expressing hJacob-SPOT. Overexpressed protein was immunoprecipitated from the cell lysate using tag specific nano-trap (SPOT-Trap-MA, Chromotek) and analysed by WB

### Immunoblotting of brain protein extracts

Human brain samples were homogenized in a buffer containing 0.32 M sucrose, 5 mM HEPES, protease inhibitors (Sigma) and phosphatase inhibitors (Roche), in a 1g/10ml ratio. The homogenates were centrifuged at 1000g for 10 min at 4°C. The pellets were used for immunoblotting. For immunoblotting of murine samples (CA1), dissected hippocampi were homogenized with TRIS buffered saline (25 mM Tris-HCI, 150mM NaCl, pH 7.4 buffer in presence of protease (Complete, Roche) and phosphatase inhibitors (PhosSTOP, Roche). Band intensities were quantified with Fiji/ImageJ (Schindelin et al., 2012).

### Quantitative real-time PCR (gPCR)

Hippocampal homogenization, RNA extraction and cDNA preparation were described previously (Spilker et al., 2016). Bdnf exon and β-actin (reference gene) cDNA were amplified using the iScript RT-PCR iQ SYBR Green Supermix (BIORAD) in a qPCR detection system (LightCycler LC480, Roche). The relative expression levels were analyzed using the 2-ΔΔCt method with normalization relative to β-actin.

### Cell based co-recruitment assay

HEK293T cells were transfected with the following constructs: Jacob-1-228 tagged with GFP with tRFP or LMO4 or LMO4 fragments (in aa): 1-165, 1-80, 81-165; PP1γ tagged with GFP (Trinkle-Mulcahy et al., 2001) and mCherry (Liu et al., 2010) or ΔMyr-Jacob tagged with tRFP. On the following day cells were fixed with 4% PFA, permeabilized with 0,1% TX-100 in 1xPBS for 10 min, stained with DAPI and mounted. Z-stack with 300 nm step size was taken with 512x512 pixels format using a Leica TCS SP8-STED system. Maximal intensity images from three optical sections were generated for representative images.

### FRET and SRET assays

For FRET experiments, HEK293T cells were transiently co-transfected with MaxPEI, (Polysciences, Cat.: #23966) with different combinations of two constructs of interest tagged with donor (GFP: LMO4, CREB, Jacob, Jacob fragments (in aa): 1-228, 262-532) or acceptor (tagRFP: LMO4, LMO4 fragments (in aa): 1-80, 81-165, CREB, CREB fragments (in aa): 1-165, 166-341), with constant concentration of cDNA of donor and increasing concentration of acceptor. FRET was performed as described previously (Carriba et al., 2008). For SRET measurements, transfected cells were resuspended in Hank's balanced salt solution (HBSS) supplemented with 10 mM glucose (Sigma-Aldrich). Triple-transfected (CREB-RIuc:Jacob-GFP:LMO4-tagRFP) cell suspension was used to perform the three measurements. (i) The LMO4-tagRFP expression level was assessed by the tagRFP fluorescence intensity. (ii) The CREB-Rluc expression level was estimated by CREB-Rluc luminescence determined 10 min after addition of coelenterazine-H (5 µM). (iii) For SRET, the cells were incubated with 5 µM of DeepBlueC (Molecular Probes). The measurements were done with Mithras LB940 (Berthold Technologies) equipped with detection filters at 400 nm and 590 nm. Net SRET was defined as [(long-wavelength emission)/(short-wavelength emission)]-Cf, where Cf corresponds to [(long-wavelength emission)/(short-wavelength emission)] for cells expressing CREB-RIuc:Jacob-GFP:LMO4-tRFP.

### Luciferase assay

HEK293T cell line stably expressing luciferase under the CRE promoter (vector pGL4.29[luc2P/CRE/Hygro], Promega) were transfected for 24h and lysed with the lysis buffer (25 mM Tris-phosphate, 2 mM DTT, 2 mM 1,2-diaminocyclohexane-N,N,N',N'-tetraacetic acid, 10% glycerol, 1% TX-100, pH 7.8). The luciferase activity was measured with the Dual-Luciferase Reporter Assay System (Promega) on a (FLUOstar Omega, BMG Labtech).

### Animal perfusion and immunohistochemistry

Animals were anesthetized with ketamine/xylazine (Medistar) and transcardially perfused with 0.9% saline followed by 4% PFA. The brains were post-fixed in 4% PFA in PBS overnight, followed by immersion in 0.5 M sucrose for 24h and then 1M sucrose for another 1 to 2 days or until the brains were sunken down. The brains were snap-frozen and cut into 35 µm thick coronal cryosections (Leica CM3050S, Leica-Microsystems). The sections were blocked in a buffer (0.3% TX-100, 10% NGS in PBS) for 30 min at RT, and incubated with primary antibodies diluted in the blocking solution, overnight at 4°C. Secondary antibodies were applied for 2h at RT. Sections were counterstained with DAPI and mounted in Mowiol 4-88 (Merck Chemicals). For the detection of Aβ, the heat-based antigen retrieval method was used. For anti-Aβ staining, following permeabilization, brain sections were immersed into 10 µM sodium citrate solution (Fluka, pH=9) for 30 min at 80°C. Imaging of nuclear pCREB/CREB immunoreactivities in cryosections was performed using a Leica TCS SP5 system (Leica-Microsystems). Images were acquired sequentially with a HCX ApoL20x/1.0 water objective, optical zoom 4. Sections were imaged with constant laser/detector settings along the z-axis with 400 nm step in a 512x512 pixel format.

### OHSC stimulation and immunocytochemistry

For Aβ-induced CREB shutoff Aβ1-42 oligomeric solution was prepared as described in (Grochowska et al., 2017). Briefly, the Aβ1-42 peptide film (Anaspec) was re-suspended in 2 µl DMSO (Sigma-Aldrich), sonicated for 5 min, diluted with F12 medium (Gibco) to final concentration of 50 µM, sonicated for 10 min, and left for oligomerization at 4°C, on. 7 DIV OHSC were treated with 1 µM of Aβ1-42 oligomers for 1h, fixed for 1h at RT in 4% PFA/4% sucrose. After fixation the slices were washed, permeabilized with 0.4% TX-100 and treated with 50 mM NH4CI for 30min. Subsequently, the samples were blocked for 1h at RT in 10% normal goat serum (NGS) in PBS. Next, the slices were incubated for 72h with primary antibody followed by incubation with the secondary antibody for 24h. Following counterstaining with 4',6-diamidino-2-phenylindole (DAPI; Vectashield/Biozol) slices were mounted in Mowiol 4-88 (Merck Chemicals).

### Primary cultures transfection and stimulation

Hippocampal neuronal cultures were transfected with plasmid cDNA using Lipofectamine2000 (Thermo Fisher Scientific) following the manufacturer's instructions. For LMO4 knockdown cells were expressing shRNA for 5 days; for Jacob knockdown 4 days. For CREB shutoff experiments hippocampal neurons were transfected at DIV15 with NLS or ΔMyr-Jacob or ΔMyr-Jacob-L175A-V176A tagged with GFP or GFP and fixed after 24h. For the experiment with okadaic acid (OA, Tocris) cells were treated for 20 min with 2 µM OA 1 day post transfection. The target sequences for LMO4 and NLS-Jacob knockdown (Spilker et al., 2016) as well as scrambled controls are indicated in key resources table. For Aβ-induced CREB shutoff experiments Aβ oligomers (Anaspec) were prepared as described previously (see previous section, (Grochowska et al., 2017)). Transfected neurons were treated with 500 nM Aβ1-42 or Aβ3(pE)-42. Nitarsone was diluted in distilled water. The concentration and duration of treatments are indicated in the figure legends.

### Immunostaining of primary neurons

Primary neuronal cultures were fixed with 4% PFA/4% sucrose solution for 10 min at RT, washed with PBS, and permeabilized with 0.2% TX-100 in PBS for 10 min. Then, cells were incubated for 1 h in blocking solution (2% glycine, 0.2% gelatin, 2% BSA, and 50 mM NH4CI (pH 7.4)) and primary antibodies were applied overnight at 4°C. Next, the coverslips were incubated with secondary antibodies diluted 1:500. Coverslips were mounted with Mowiol 4-88 (Merck Chemicals). For detection of Jacob-CREB and Jacob-LMO4 co-localization in STED imaging, a heat-based antigen retrieval protocol was used.

For surface expression of AMPA receptors dissociated hippocampal neurons were incubated for 10 min at RT with anti-GluA1 antibody diluted in Tyrode's buffer (128 mM NaCl, 5 mM KCI, 1 mM MgCI2, 2 mM CaCI2, 4.2 mM NaHCo3, 15 mM HEPES, 20 mM glucose, pH 7.2-7.4), rinsed, and fixed for 10 min at RT with 4% PFA-sucrose, and subsequently stained with other antibodies as described above.

### STED microscopy

STED images were obtained using a Leica TCS SP8 STED 3X equipped with pulsed White Light Laser (WLL) and diode 405 nm for excitation and pulsed depletion laser 775 nm and Leica HC ApoCS2 100×/1.40 oil objective. MAP2 and DAPI were acquired in confocal mode. The pixel size for all images was 20-23 nm (XY-plane). The Z-step was 150 nm. STED and confocal images were deconvolved using Deconvolution wizard (Huygens Professional, SVI), with optimized iteration method. Intensity profiles were created in Fiji/ImageJ.

### Neuronal nuclei isolation and flow cytometry

Nuclei were isolated with a Nuclei Isolation Kit (Sigma Aldrich) according to the manufacturer's protocol. Nuclei were fixed with ice-cold methanol for 10 min. 0.5% Triton for permeabilization and 10% normal donkey's serum for blocking were used followed by 1h RT incubation with respective primary and, subsequently, secondary antibodies (see key resources table). Nuclei were counterstained with Hoechst33342 (1:500, ThermoFisher Scientific). Samples were measured with a BD LSR II flow cytometer (BD Biosciences) and analysed with FlowJo (LLC).

### Whole-cell voltage-clamp recording of mEPSCs

mEPSCs of hippocampal primary neurons (DIV16-20) were recorded in the whole-cell voltage-clamp mode using 3-8 MΩ pipettes filled with an intracellular solution containing: 115 mM Cesium-methanosulfonate, 10 mM CsCI, 5 mM NaCl, 1 mM CaCI2, 2 mM MgCI2, 2 mM EGTA-acid, 10 mM HEPES, 4 mM Mg2+-ATP, 0.4 mM Na+-GTP, pH 7.4 with CsOH (290 mOsm). To block spontaneous action potential generation and GABAA receptors, 0.5 µM tetrodotoxin (TTX, Tocris) and 5 µM bicuculline (Tocris) were routinely added to the extracellular solution containing: 140 mM NaCl, 5 mM KCl, 2 mM CaCI2, 0.8 mM MgCI2, 10 mM HEPES, 10 mM glucose, pH 7.4 with NaOH (300 mOsm). Neurons were transferred to a RC26 GLP recording chamber (Warner Instruments), placed on a Leica TCS SP2 microscope equipped with 40X water objective, and perfused with extracellular solution at a rate of 1 ml/min at RT. Axopatch 200B (Molecular Devices) amplifier controlled with HEKA Patchmaster software (HEKA Elektronik GmbH) was used for recording the mEPSCs current traces at RT. Membrane potential was held at -60 mV, and current traces were filtered at 2 kHz low-pass basal filter and digitised at 10 kHz with Digidata 1440A (Molecular Devices). Current traces of continuous recordings were followed by offline analysis using Mini Analysis 6 software. Only whole-cell voltage-clamp recordings from cells with a holding current of < 200 pA were included in the analysis.

### Acute hippocampal slices and LTP recordings

The 350 µm thick hippocampal slices were prepared according to a previously described protocol (Grochowska et al., 2017). Briefly, hippocampal slices were incubated in bubbling 95% O2, 5% CO2 ASCF solution for 2 h at 31 +/- 1°C. Field excitatory postsynaptic potentials (fEPSPs) were measured in stratum radiatum after stimulation of CA1 Schaffer collateral fibers with ACSF glass capillary microelectrodes (3-5 MΩ). fEPSPs were amplified by Extracellular Amplifier (EXT-02B, npi, Germany) and digitized at a sample frequency of 5 kHz by Digidata 1201 plus AD/Da converter (CED, UK). The strength of stimuli was adjusted to 25-35% of the maximum fEPSP slope values. Single stimuli were applied at 0.0333 Hz and averaged every 5min. Stable baseline recordings were followed by tetanisation with 3×1s stimulus trains at 100 Hz with a 10 min inter-train interval.

### SPECT-imaging of cerebral blood flow

Mice (three months of age, both sexes) were intravenously injected with the lipophilic 99mTc-D,L-hexamethylene-propyleneamine oxime (99mTc-HMPAO) via chronically implanted catheters in the right external jugular vein. Jugular vein catheter implantation and preparation of the 99mTcHMPAO injection solution were done as described in (Kolodziej et al., 2014). For tracer injection, the catheter was extended by a polyethylene (PE) tube (BioMedical Instruments, 60 cm, prefilled with 150 µl 0.9%NaCl) and connected to a syringe containing 130-170MBq/330 µl of freshly prepared 99mTc-HMPAO-solution. Injections were made at flow rates of 33 µl/min during periods of 15 min. During this time, the animals were awake and freely moving. After injection, animals were anaesthetized (4-1.5% isoflurane, 800 ml/min O2) and transferred to the single photon emission computed tomography/Computed Tomography (SPECT/CT)-scanner. The injected 99mTc activity was calculated by determining the amounts of 99mTc that had remained in the syringe and in the extension tube by using a radionuclide calibrator (Aktivimeter Isomed 2010, Nuklear-Medizin-Technik Dresden GmbH). Co-registered head SPECT/CT-scans were made using a four-head NanoSPECT/CT (Mediso). CT scans were made at 45kVp, 177µA, with 180 projections, 500 ms per projection and 96 µm spatial resolutions, reconstructed (InVivoScope 1.43) at isotropic voxel-sizes of 100 µm. SPECT scans were made using ten-pinhole mouse brain apertures with 1.0 mm pinhole diameters, providing a nominal resolution of <0.7 mm. Twenty-four projections were acquired during a scan time of one hour. Axial FOV was 20.9 mm. Photopeaks were set to the default values for 99mTc (140keV +/- 5%). SPECT images were reconstructed (HiSPECT, v 1.4.1876, SCIVIS) at an isotropic voxel output size of 250 µm. The co-registered SPECT/CT-images were aligned to a reference MR (MPI Tool software, v6.36, ATV; Dorr-Steadman-Ullmann-Richards-Qiu-Egan (40 micron, DSURQE)) and SPECT brain data-sets were global mean normalized using ImageJ. Further data processing and calculation of group means and differences and statistical analyses were done using ImageJ and Matlab (R2017b). Results were illustrated with Osirix (v. 5.8.1) (Rosset et al., 2004).

### Nitarsone feeding regime

Nitarsone (98%, ABCR Gute Chemie) was prepared in aliquots of 15 mg/850 µl (350 µl dH₂O, 500 µl MediGel Sucralose, Clear H₂O) to be used within five days. Mice were treated with Nitarsone (50 mg/kg body weight) or vehicle (sucrose solution) once a day per oz for a total of 6 to 7 weeks. For TBA2.1 mice (both sexes), treatment started at the age of 4 weeks. The animals were group housed and force fed for 4 weeks. Afterwards they were single housed and allowed to choose voluntary feeding (Nitarsone or vehicle mixed into a gel paste, DietGel boost Hazelnut, ClearH₂O) over continued force-feeding. Treatment in 5xFAD male mice started at the age of 12 weeks. Here, animals were single housed right away and given a choice between voluntary and forced feeding. Behavioral experiments were conducted for both lines during the 6th week of Nitarsone treatment. During the course of the 7th week of treatment, animals were either subjected to perfusion to obtain their brains for subsequent immunohistochemistry or they were culled to obtained native brains for electrophysiological recordings.

### Open-field test

Locomotor activity was assessed using the Open-field test, performed in a square arena (45x45x45cm) made of black Plexiglas and dimly illuminated. The animals were placed in the center of the arena, and were left to explore it for 10 min. The sessions were video recorded, and the distance travelled in the maze as well as the speed was tracked with ANY-maze Software 7.0 (Stoelting Co). Data were analyzed in 1 min bins.

### Novel object recognition and location

The hippocampus-dependent memory was assessed using a novel object location and novel object recognition tasks. Assays were performed as described elsewhere (Andres-Alonso et al., 2019). Briefly, training and testing were done in an open field square arena made out of plastic (50 cm × 50 cm). The mice were habituated in the empty arena for 20 min. Subsequently, 2 identical objects were introduced to the maze and the animals could explore the objects in 2 training session, 20 min each. 2 h afterwards, in the memory test phase, one object was replaced with an unfamiliar, unknown object (novel object recognition). Afterwards, the location of one object was changed (novel object recognition). During all sessions, the amount of time the animals explored the objects was recorded, and a discrimination index was calculated using the formula DI=[(Tnew-Tfamiliar)/ (Tnew+Tfamiliar)]*100. The arena was cleaned with 5% ethanol before and after each animal was tested.

### Y-maze Object Recognition

Y-maze Object Recognition short-term memory task was performed according to (Creighton et al., 2019). During training session, two identical objects (3D printed rectangle) were placed at the end of arms B and C and the animal was left to explore both objects for 10 min. In order to assess short-term memory, retrieval performance was tested 3 h after training with one of the familiar objects replaced by a novel one (wooden cube). The mice were placed in the arena, and had 5min to explore both objects. During both sessions the time animals explored the objects was scored, and a discrimination index was calculated, in the same manner as for the novel object recognition test.

### Results

### CREB shutoff and reduced levels of phosphorylated Jacob in brains of AD patients

The levels of Jacob phosphorylated at S180 (pJacob) and total Jacob in post-mortem tissue of AD patients were first examined (see Table 1 for information on patients). Immunoblotting of a nuclear enriched fraction obtained from the temporal cortex of AD patients did not reveal a significant reduction in total Jacob levels as compared to controls (Fig. 7a-c). However, the levels of pJacob were significantly decreased by roughly 40% (Fig. 1a, b; Fig. 7a), indicating that nuclear import of Jacob following activation of synaptic NMDAR is diminished probably at the expense of activation of extrasynaptic NMDAR (Karpova et al., 2013). The inventors observed significant neuronal loss in AD patients as evidenced by NeuN-immunoblotting (Fig. 7d, e). Since Jacob, unlike CREB, is exclusively expressed in neurons (Mikhaylova et al., 2014), the inventors could compare phosphorylation of nuclear Jacob normalized to total protein levels and corrected these values for NeuN content to adjust for neuronal cell loss (Fig. 1c). With this measure the inventors also observed a clear reduction of the pJacob/Jacob ratio (Fig. 1c). This in turn was correlated with the degree of CREB shutoff that we had to determine following FACS sorting of NeuN-positive nuclei (Fig. 1d-g, Fig. 7f), given that CREB is expressed in both, glia cells and neurons. Collectively, these data show CREB shutoff in AD brains and a correlation with lower levels of pJacob that in turn suggests a functional link.

### Jacob protein knockdown and gene knockout protects against Aβ toxicity

Aβ oligomers can be found in various, post-translationally modified forms, out of which the N-terminally truncated, pyroglutamylated Aβ3(pE)-42 species are prominent in the brain of AD patients (Bayer and Wirths, 2014; Kummer and Heneka, 2014). Previous work suggests that Jacob plays a role in Aβ-induced CREB shutoff that is elicited by activation of extrasynaptic GluN2B containing NMDAR (Gomes et al., 2014; Grochowska et al., 2017; Rönicke et al., 2011). shRNA knockdown of Jacob in hippocampal neurons indeed prevented CREB shutoff induced by treatment of cultures with 500 nM Aβ1-42 or Aβ3(pE)-42 oligomers (Fig. 7g, h). Similar results were obtained in organotypic hippocampal slices from Jacob knockout mice or wild-type littermates treated with 1 µM oligomeric Aβ1-42 (Fig. 1g, h). Basal pCREB immunofluorescence levels were not different between both genotypes, however, neurons from knockout mice, unlike the wild-type, did not display Aβ1-42-induced CREB shutoff (Fig. 1g, h). Total CREB levels remained unchanged (Fig. 2i, j).

### Jacob gene knockout ameliorates neuronal loss in transgenic AD mice

The inventors next reasoned that the lack of Aβ-induced CREB shutoff in Jacob knockout mice could confer neuroprotection in AD. The CA1 subfield of the hippocampus is one of the areas earliest affected in AD, with pronounced neuronal loss and a decreased number of synaptic contacts (Padurariu et al., 2012; Price et al., 2001; Wirths and Zampar, 2020; Yiu et al., 2011). TBA2.1 mice express Aβ3(pE)-42 and display severe CA1 neuronal loss, amyloidosis, LTP impairment, and neuroinflammation (Alexandru et al., 2011). Western blot analysis of protein extracts from TBA2.1 mice revealed that, while Jacob protein levels remained unchanged (Fig. 7i, j), pJacob levels are decreased resulting in a reduced pJacob/Jacob ratio like in human brain (Fig. 7j-l). In accordance with reports from other AD transgenic mouse lines (Bartolotti et al., 2016; Caccamo et al., 2010; Yiu et al., 2011), we found that TBA2.1 mice exhibit significantly reduced nuclear pCREB levels (Fig. 1k, l). To directly study whether the loss of Jacob expression in neurons confers neuroprotection in TBA2.1 mice the inventors next crossed both lines to obtain homozygous TBA2.1 and Jacob/Nsmf knockout (-/-) mice. Interestingly, the double transgenic animals (TBA2.1 x Jacob/Nsmf -/-) did not display CREB shutoff (Fig. 1m, n). Although we inventors observed in all three genotypes (TBA2.1, Jacob/ Nsmf -/-, and double TBA2.1 - Jacob/Nsmf -/-) slightly decreased nuclear CREB levels (Fig. 1m, n), the lack of CREB shutoff in knockout and double transgenic animals points to a key role of Jacob in CREB shutoff at an early stage of Aβ-amyloidosis. Accordingly, cell-loss in the dorsal CA1 region was less pronounced in double transgenic mice (on average 23%) (Fig.1o). The rescue mediated by Jacob knockout was also visible at the level of brain-wide network activation patterns when the inventors imaged cerebral blood flow (CBF) in unrestrained behaving mice of all four genotypes (TBA2.1; Jacob/Nsmf -/-; TBA2.1, Jacob/Nsmf -/-; - /-, and wild-type) using SPECT (Kolodziej et al., 2014; Oelschlegel and Goldschmidt, 2020). Decreases in CBF, found in dorsal CA1 (arrow) of TBA2.1 mice when compared to wild type animals, were partially rescued in double transgenic mice (Fig. 1p). This rescue was also visible in the lateral septum and the diagonal band, regions connected to the hippocampus (arrows; Fig. 7m). In addition, mRNA levels of BdnfIV, a synaptic plasticity related neurotrophic factor (Spilker et al., 2016), whose expression is regulated by CREB in an activity-dependent manner, were decreased in TBA2.1 mice, but not in Jacob/Nsmf (-/-) and double transgenic animals (Fig. 1r). Most important, Jacob gene deletion did not influence the number of astrocytes (Fig. 7n, o) and activated microglia (Fig. 7o, p). Moreover, amyloid load, evidenced by the number of Aβ-positive deposits, (Fig. 7r, s) was not affected as well, indicating that indirect effects of neuroinflammation or amyloid deposition will not account for the neuroprotection conferred by Jacob gene deletion.

### Jacob is a direct binding partner of CREB and LMO4

The inventors next aimed to decipher the underlying molecular mechanisms of Jacob-induced CREB shutoff. A pull-down assay with bacterially expressed proteins revealed a direct association of both N-terminal 117-172 amino acid (aa) and C-terminal (262-532 aa) regions of Jacob to the bZIP domain of CREB (Fig. 2a / Fig. 8a-c). Accordingly, super-resolution stimulated emission depletion (STED) imaging showed nuclear Jacob in close proximity to CREB in cultured hippocampal neurons (Fig. 8d, e). The inventors could co-immunoprecipitate endogenous CREB from HEK293T cells following heterologous expression of Jacob (Fig. 8f) and in support of these data the inventors found prominent in vivo FRET efficiency when the inventors co-expressed either full-length or the N-terminal half of Jacob and a C-terminal fragment of CREB (Fig. 2b-e). Of note, the N-terminal fragment of Jacob yielded significantly stronger fluorescence resonance energy transfer (FRET) signals than the C-terminal fragment (Fig. 2b-e). In a yeast two-hybrid (YTH) screen performed with the N-terminus of Jacob as bait the inventors identified LMO4 as a binding partner (Fig. 9a). LMO4 is a transcriptional co-activator of CREB (Kashani et al., 2006) and the inventors therefore analyzed whether the Jacob-LMO4 interaction has a role in Jacob-induced CREB shutoff. A region encompassing aa 117-228 of Jacob interacts with the LIM1 domain of LMO4 (Fig. 9a). Pull-down assays showed direct binding (Fig. 2f, Fig. 9b, c). Both proteins are in close proximity to each other in neuronal nuclei (Fig. 9d, e) and the LIM1 domain co-localizes with cytosolic Jacob clusters following heterologous expression (Fig. 9f). The association of both proteins was further confirmed by heterologous co-immunoprecipitation with tag-specific antibodies (Fig. 9g) and a direct interaction was corroborated by in vivo FRET analysis (Fig. 3g-i). Heterologous co-immunoprecipitation of Jacob fragments with the LIM1 domain indicates that the binding regions for LMO4 and CREB interactions do not overlap (Fig. 3j). Moreover, like other LIM1 domain binding proteins, Jacob contains a leucine- and valine rich stretch (Joseph et al., 2014) and point mutations (L175A-V176A, Fig. 2k) within this region resulted in much weaker binding (Fig. 2k-m). Nuclear overexpression of non-phosphorylated Jacob leads to dephosphorylation of CREB (Dieterich et al., 2008). Interestingly, nuclear accumulation of the LMO4 binding mutant of Jacob did not induce CREB shutoff (Fig. 2n-p), indicating that the association with LMO4 is instrumental for Jacob-induced dephosphorylation of CREB.

### Jacob competes with CREB for LMO4 binding

The inventors next asked why the association with LMO4 is crucial for Jacob-induced CREB shutoff. In vivo FRET assays and heterologous co-immunoprecipitation revealed that the LIM1 domain, which is the binding interface for Jacob (Fig. 2 i, j, Fig. 8a, f, g), also interacts with a C-terminal fragment of CREB (Fig. 3a-c). LMO4 directly binds to this region but not like Jacob to the isolated bZIP domain of CREB (Fig. 3d-f, Fig. 9a-c). It was found that the direct interaction with LMO4 promotes phosphorylation of S133 and thereby CREB transcriptional activity as evidenced by increased CRE-driven luciferase activity following heterologous expression of LMO4 (Fig. 3g). shRNA knockdown of LMO4 (Fig. 9h-j) in neurons resulted in reduced pCREB (Fig. 3h, i) but not total CREB immunofluorescence (Fig. 3j, k). Direct binding of LMO4 to CREB and Jacob raised the question whether all three proteins can assemble in a trimeric complex or whether they compete for the same binding interface. Sequential resonance energy transfer (SRET) in vivo indeed revealed the existence of a triple complex (Fig. 31, m). Jacob harbors a binding interface for the interaction with CREB and LMO4 at the N-terminus (Fig. 2) whereas LMO4 can only associate through the first LIM1 domain either to Jacob or to CREB (Fig. 2, 3). Subsequent competition pull-down experiments confirmed competitive binding between the N-terminus of Jacob and CREB with LMO4 when GST-LMO4 was coupled to the beads and increasing amounts of the N-terminal fragment of Jacob were added in the presence of CREB (Fig. 3n-p). In summary, the LIM1 domain of LMO4 mediates either the association with CREB or Jacob and Jacob is capable to displace LMO4 from the CREB complex, a mechanism that should facilitate CREB shutoff.

### PP1γ and LMO4 are involved in Jacob-induced CREB shutoff

Previous work has shown the involvement of protein phosphatase 1 (PP1) in NMDA-induced CREB shutoff (Sala et al., 2000). Jacob harbors several PP1 binding motifs, both proteins co-localize following heterologous expression (Fig. 9k) and tagged Jacob co-immunoprecipitates with endogenous PP1γ (Fig. 91). Pull-down experiments established a direct interaction (Fig. 4a, Fig. 9m) and heterologous co-immunoprecipitation experiments revealed two binding interfaces (Fig. 4b). The inventors therefore next addressed whether the association with PP1γ is involved in Jacob-induced CREB shutoff. To this end the inventors expressed a phosphodeficient mutant of Jacob in the nucleus of hippocampal primary neurons (Karpova et al., 2013) and incubated cultures with the PP1γ inhibitor okadaic acid (OA). Interestingly, it was found that treatment with OA indeed prevented Jacob-induced CREB shutoff (Fig. 4c, d). Phosphorylation of Jacob at S180 is induced by activation of synaptic NMDAR, whereas nuclear import of non-phosphorylated Jacob is related to extrasynaptic NMDAR activation (Karpova et al., 2013). Accordingly, in a CRE-luciferase activity assay the expression of wild-type but not phosphodeficient Jacob caused increased activity (Fig. 4e, Fig. 9n). Furthermore, the association of a phosphodeficient mutant of Jacob is significantly stronger with LMO4 than the corresponding phosphomimetic protein (Fig. 4f, g, Fig. 9o, p). Phosphomimetic Jacob, unlike the non-phosphorylated protein, did not displace CREB from LMO4 bound to beads (Fig. 4h, i, Fig. 3n-p). To further test the idea that LMO4 binds to non-phosphorylated Jacob more efficiently the inventors applied the protein kinase inhibitor staurosporine and performed heterologous co-immunoprecipitation experiments (Fig. 4j, k). They indeed found a stronger association of non-phosphorylated Jacob to LMO4 (Fig. 4j, k) and concomitantly a stronger association of S180 phosphorylated Jacob to CREB (Fig. 4j, k). Since Jacob directly interacts with PP1γ (Fig. 4a) the inventors performed a pull-down assay where they observed that the association between both proteins was much stronger in the presence of LMO4 (Fig. 41, m, Fig. 10o, p). Thus, non-phosphorylated Jacob, entering the nucleus following activation of extrasynaptic NMDAR, will likely displace LMO4 from the CREB complex and the subsequent association with LMO4 will enhance binding to PP1γ, which then ultimately results in CREB shutoff.

### The small organoarsenic compound nitarsone selectively blocks binding of Jacob but not of CREB to the LIM1 domain of LMO4

The molecular analysis outlined above allowed the inventors to perform structural modeling of the binding interface between CREB, Jacob and the LIM1 domain of LMO4. To this end we analyzed deposited peptide-bound LMO4 structures. In LMO4:LIM domain-binding protein 1 (Ldb1) (Deane et al., 2004) a peptide of Ldb1 binds to LMO4 by short β-β main chain formations and single hydrophobic side chains protruding into deep pockets in each of the two LIM domains (Fig. 5a). LMO4:Ldb1 highlights the relatively weak sequence preferences for peptides binding to either or both LIM domains (Fig. 5a). Therefore, the inventors performed a positional scan by computational serial mutation of each peptide residue to any of 20 aa stretches in both Jacob and CREB which let them define a search pattern of only 4 critical residues for potential LMO4 binding regions (Fig. 10a-d). With this approach the inventors found eight potential binding regions in Jacob and five in CREB (Fig. 10b), for which they modeled LIM1:peptide complexes and calculated complex stability. For Jacob the inventors confirmed a peptide including residues 172-186 that is part of the experimentally localized LMO4 binding region. In addition, the inventors found two overlapping peptides within the experimentally determined LMO4 binding region for CREB (Fig. 10d). The inventors next searched for small molecules that might selectively prevent binding of Jacob and not of CREB to LIM1 of LMO4. Here, the inventors used ZINCPharmer (Koes and Camacho, 2012), a tool that allows to define donor and acceptor atoms within the hydrophobic binding pocket (Fig. 5b; SEQ ID NO 4, P61968, LMO4_Human) and the β-strand (β3, Fig. 5c) of LIM1 (Koes and Camacho, 2012). Several hits contained a p-nitrophenyl group (e.g. 2-[[1-(4-nitrophenyl)ethyl]amino]ethan-1-ol (ZINC37177221)) fitting to the hydrophobic binding pocket. The inventors therefore next searched the Drugbank database (Wishart et al., 2006) for purchasable drugs containing this group and identified p-nitrophenylarsonic acid (Nitarsone) as promising candidate since Nitarsone fitted into the hydrophobic binding pocket of LIM1 as evidenced by AutoDock Vina (Eberhardt et al., 2021) (Fig. 5d, SEQ ID NO 5, P61968, LMO4_Human). To prove efficacy, specificity and affinity of Nitarsone binding to the LIM1 domain the inventors next purified recombinant proteins expressed in bacteria (Fig. 10e). Isothermal titration calorimetry revealed a single binding site in LMO4 and a KD of 0.77 µM (Fig. 5e), which is matching the KD of 0.37 µM for binding of Jacob to LMO4 (Fig. 5f). These results prompted the inventors to test the prediction that Nitarsone will only block binding of LMO4 to Jacob but not to CREB. In GST-pulldown experiments the inventors could show that Nitarsone completely abolished binding of Jacob to LMO4 when applied in 5x times molar excess (Fig. 5g-i; Fig. 10e). In these experiments we immobilized 500 nM GST-LMO4, saturated binding with 1 µM Jacob 45-228 and then applied 5 µM Nitarsone. However, even at a concentration of 20 µM, i.e. 20x times molar excess, the substance did not displace CREB from LMO4 (Fig. 5j-l, Fig. 10e).

### Nitarsone application rescues Aβ-induced CREB shutoff as well as synapse loss and synaptic dysfunction

The inventors next found that bath application of 10 µM Nitarsone prevented acute Aβ-induced CREB shutoff in hippocampal primary neurons (Fig. 10f-h). The drug was either applied 30 min prior or 2h after application of 500 nM Aβ for 2h. In both conditions the inventors found a rescue of pCREB levels following Nitarsone administration (Fig. 10f-h). Co-application of an even lower dose of 5 µM Nitarsone also rescued CREB shutoff induced by application of 500 nM Aβ oligomers for 48 h (Fig. 5m, n). The inventors therefore next assessed Aβ-induced synapse loss in dissociated hippocampal neurons that were kept for 48 h in the presence of 5 µM Nitarsone (Fig. 5o, p). In control experiments these neurons exhibited normal up-scaling of GluA1 AMPA-receptors in response to silencing of neuronal activity with 1 µM TTX application (Fig. 10i). Aβ induced a 30% reduction of synaptophysin/Shank3 puncta in these cultures (Fig. 5o, p) and this synapse loss was completely prevented by Nitarsone application (Fig. 5o, p). The concomitant downscaling of synaptic surface expression of GluA1 AMPA-receptors was also significantly attenuated in the presence of Nitarsone (Fig. 5r, s). Whole-cell patch-clamp experiments showed that reduced surface expression of GluA1 following Aβ treatment was accompanied by reduced miniature excitatory postsynaptic current (mEPSC) amplitude but not frequency (Fig. 5t-x). Co-application of 5 µM Nitarsone restored mEPSC amplitude while administration of the drug alone had no effect on both measures (Fig. 5t-x).

### In vivo administration of Nitarsone prevents early synaptic dysfunction and cognitive deficits in two transgenic AD mouse lines

The inventors next administered Nitarsone *in vivo* in two transgenic AD mouse lines with amyloid pathology, TBA2.1 and 5xFAD mice. 5xFAD mice express human APP and PSEN1 transgenes with a total of five AD-linked mutations (Oakley et al., 2006). These mice display less rapid spread of amyloid pathology than TBA2.1 mice, with visible plaques accompanied by gliosis at four months of age with accompanying synaptic dysfunction and cognitive impairment (Mikhaylova et al., 2014). The inventors administered Nitarsone orally with forced feeding and a defined daily dose of 50 mg/kg that was based on a conservative NOEL (no observed effect level) from several toxicology studies and the rationale to achieve an effective dose in brain tissues (see Fig. 11a for experimental details of administration). Accordingly, this regime had no effect on the body weight of treated as compared to control animals, implicating that no toxicity of the treatment occurred . In addition, the inventors did not detect any effects of Nitarsone treatment on amyloid load in both mouse lines (Fig. 6j-m). Nitarsone administration effectively prevented CREB shutoff in both transgenic AD mouse lines in the dorsal hippocampal CA1 region following 6 weeks of treatment (Fig. 6a-h). In addition, early neuronal cell loss was reduced in TBA2.1 mice in comparison to vehicle-treated littermates (Fig. 6i, Fig. Fig. 1o, 11d,e). The inventors could not detect neuronal loss in CA1 in 5xFAD mice at 19 weeks of age (Fig. 11d, e). In addition, the inventors found clearly reduced synapse loss in the stratum lacunosum moleculare in mice treated with Nitarsone in both animal models of amyloid pathology (Fig. 6j-m). Interestingly, this is the first stratum affected by amyloid-pathology in many AD animal models (Kerchner et al., 2012; Su et al., 2018). In accord, with these findings the inventors observed early synaptic dysfunction in acute hippocampal slices, as evidenced by deficits in late-phase long-term potentiation (LTP), in TBA2.1 and 5xFAD mice at postnatal week 11 and 19, respectively (Fig. 6n-q). The reduced fEPSP slope in the last 30 minutes of recordings could was rescued in slices of Nitarsone-fed mice in both AD lines (Fig. 6n-q), indicating a rescue of synaptic plasticity that is relevant for learning and memory (Fig. 6n-q, Fig. 11f, g). The inventors therefore next determined whether treatment with Nitarsone also rescues cognitive decline in TBA2.1 and 5xFAD mice (Alexandru et al., 2011; Oakley et al., 2006). To evaluate short-term memory, they used the Y-maze object recognition task (Creighton et al., 2019), which minimizes contextual cues, with an interval of 3h between training and test. Object recognition was impaired in TBA2.1 and 5xFAD mice treated with vehicle, when compared to littermate controls (Fig. 6q, r). Conversely, transgenic TBA2.1 and 5xFAD mice fed with Nitarsone displayed improved discrimination performance in comparison to vehicle treated animals (Fig. 6q, r). Human AD patients display impairments in object recognition tasks which essentially rely on proper synaptic function of CA1 neurons (Didic et al., 2013). Accordingly, TBA2.1 and 5xFAD mice showed deficits in novel object location and novel object recognition memory (Fig. 6s-v, Fig. 11 l-o). Treatment of TBA2.1 and 5xFAD animals with Nitarsone also rescued memory in a novel location recognition as well as in a novel object recognition task with a cognitive performance comparable to vehicle-treated littermate controls (Fig. 6s-v). Collectively, these data provide evidence that restoring synaptic plasticity with Nitarsone improve hippocampus-dependent learning and memory despite the presence of manifest amyloid pathology.

### Discussion

Several lines of evidence suggest that the CA1 region, in both human patients and mouse AD models, is among the first to exhibit deficits in CREB activation, synaptic function and neuronal excitability. Despite this central role of CREB, research on amyloid pathology was largely focused on local signaling events that acutely elicit decay of synaptic function, largely ignoring the fact that molecular mechanisms underlying inactivation of CREB in AD remained elusive. Herein the inventors revealed a molecular mechanism implying Aβ-induced extrasynaptic NMDAR activation and nuclear import of Jacob for the induction of CREB shutoff. Molecular modeling and screening for small chemical molecules subsequently led to the surprising discovery that nitarsone blocks binding of Jacob to the LIM1 domain. Unexpectedly, application of Nitarsone *in vitro* and *in vivo* proved the relevance of the deciphered molecular mechanism for Aβ-induced synaptic pathology, CREB shutoff and the progression of synaptic and cognitive dysfunction at the early stage of AD. Taken together, the inventors surprisingly found support that macromolecular protein transport to the nucleus has a pathophysiological role in amyloid-pathology. No other molecular mechanism for long-lasting transcriptional inactivation of CREB in neurons has been described yet and it is shown by the findings of the inventors that this mechanism will also contribute to early synaptic dysfunction elicited by similar mechanisms in other slowly progressing neurodegenerative diseases.

### A molecular mechanism for CREB shutoff in AD

The inventors herein provide evidence that Jacob directly associates with the bZIP domain of CREB and they could further show that binding of Jacob to either LMO4 or α-internexin determines whether Jacob associates with the CREB phosphatase PP1γ or the kinase ERK1/2 and binding to either of these adaptor proteins is decisive whether Jacob induces inactivation of CREB or enhanced CREB-dependent gene transcription. LMO4 is a transcriptional co-activator of CREB (Kashani et al., 2006) and the data presented herein suggests that LMO4 will hinder dephosphorylation of S133, stabilize the CREB dimer and thereby act as a transcriptional enhancer. Jacob likely displaces LMO4 from the CREB complex (Fig. 3n-p), and the inventors consider that this contributes to long-lasting CREB dephosphorylation. Thus, enhanced binding of Jacob to PP1γ and displacement of LMO4, renders the association with LMO4 a key event for Jacob-induced CREB shutoff. PP1γ phosphatase activity is regulated by a large and growing number of targeting subunits, as well as by a smaller number of inhibitor proteins that interact with PP1γ in a mutually exclusive manner (Bollen et al., 2010). In this regard the inventors contemplated that Jacob could displace an inhibitor from PP1γ and thereby regulate its activity or whether it serves exclusively as a targeting subunit.

### CREB shutoff and the Jacob-signalosome contribute to early synaptic dysfunction in AD

Collectively the data shown in the present Example evidences that long-distance protein transport from NMDAR to the nucleus is an important mechanism for disease progression at an early stage in AD. The inventors contemplate that this stage follows the initial hyperexcitability that has been described in transgenic mice with Aβ pathology (Busche et al., 2012; Lam et al., 2017; Li and Selkoe, 2020). Recent work suggests that this hyperexcitability is at least in part caused by the suppression of glutamate reuptake (Zott et al., 2019), which in turn might cause sustained activation of extrasynaptic NMDAR in response to increased ambient glutamate levels. The inventors contemplate that Jacob-induced CREB shutoff kicks in when extrasynaptic NMDAR activation is continuous and further driven by agents like oligomeric Aβ. In addition, the inventors propose that nuclear import of Jacob might be the initial trigger for decay of synaptic function that is induced by altered gene transcription. The most promising therapeutic window in AD is right at the beginning of synaptic dysfunction, and in light of the present study Jacob is an attractive target for interventions to rescue or even restore synaptic plasticity. Interestingly, the improvement in spatial memory appeared to occur independently of Aβ plaque load and might be related to the extent of synapse loss, which is a more robust correlate of cognitive impairment in AD patients at an early stage than Aβ or neurofibrillary tangle deposition. The intervention with nitarsone, most importantly, opens up new and much more selective therapeutic avenues that directly target altered NMDAR-to-nucleus communication at the onset of AD. Nitarsone selectively interrupts the interaction of Jacob but not of CREB to the LIM1 domain of LMO4 and competes with a 15 amino acid short peptide in Jacob that binds to LIM1. Moreover, the inventors identified two peptides within the LMO4 binding region of CREB. Structural modeling predicts that CREB binds with both peptides to a LIM domain tandem of LMO4 with similar binding energies as Ldb1, but two-times higher than Jacob (Fig. 10a-d). The second LIM2 domain has a weaker binding site for peptides than LIM1 as already shown for CtIP and Lbd1 (Deane et al., 2004; Stokes et al., 2013). Of note, according to the inventors model CREB-binding may not interfere with self-association of the LIM2 domain of LMO4.

### The therapeutic potential of Nitarsone

Nitarsone has been in use in poultry farming as a food additive to prevent histomoniasis and to improve food utilization. A potential health risk for a human consumer by nitarsone was evaluated to be very low, where in an estimated life-long consumption of turkey meat might result in increased lifetime risk of developing cancer of 0.00031% (Nachman Keeve et al., 2017; Nachman et al. (https://doi.org/10.1289/EHP225).

Nitarsone is the oxidized form of arsanilic acid, an organic arsenic compound, considered to be less harmful than inorganic arsenic or arsenic trioxide (ATO) (Fowler et al., 2022). In this regard it should be mentioned although human arsenic methyltransferases in liver convert ATO to cytotoxic arsenic (Maimaitiyiming et al., 2020), ATO has become the standard treatment of acute promyelocytic leukemia (de Almeida et al., 2021; Lo-Coco et al., 2013). The dose that was used in the present Example should be well tolerated in humans and the regular consumption of so-called arsenic eater has reportedly no detrimental health effect. In fact, arsenic has a long tradition in folk and veterinary medicine and was used for many years to treat syphilis and other disease states (Iland and Seymour, 2013). In light of these arguments and given that AD, PD or dementia are lethal neurodegenerative diseases starting usually at higher age, the inventors consider Nitarsone a reasonable therapeutic option to attenuate early synaptic dysfunction and cognitive decline and thereby to slow down disease progression.

### Image analysis of murine brain sections

All quantifications were done within the distal CA1 region of the hippocampus. Fiji/ImageJ software (Schindelin et al., 2012) was used to calculate maximum intensity projection from five optical sections for each channel. CREB and pCREB immunoreactivity of every single nucleus in a 100 µm stretch was measured in arbitrary units of pixel intensity. ROIs were defined based on NeuN and DAPI. The neuronal loss was quantified based on NeuN staining (the number of neurons/CA1 length). For the quantification of microglia and astrocytes the number of cells based on overlaid DAPI and Iba-1 or GFAP signal was quantified within the rectangular ROI. Aβ plaques were counted in the region where the neuronal loss was quantified.

### Image analysis of hippocampal cultures

For quantitation of nuclear staining intensity (pCREB or CREB) somatic regions were sequentially scanned using the 63x objective (Leica) in both: Leica TCS SP8-STED and Leica TCS SP5 systems. Image format was set either to 1024x1024 or 512x512 with optical zoom 4. Average intensity images from three optical sections (z-step 300 nm) were generated and intensity measurements were performed within the ROIs defined by DAPI. The values were normalized to the mean intensity of the control group. Unprocessed images were analysed using Fiji/ImageJ software (Schindelin et al., 2012). For visualization of the quantified channel a fire look-up table (LUT) was used. Synaptic density was quantified in secondary dendrites using Fiji/ImageJ (Schindelin et al., 2012). The number of synaptophysin and Shank3-positive puncta was divided by the length of the dendritic segment. Intensity of the GluA1 channel was measured in ROIs defined by a Shank3 mask as a readout for surface AMPAR expression.

### REFERENCES

Behnisch T, Yuanxiang P, et al. (2011) Nuclear translocation of jacob in hippocampal neurons after stimuli inducing long-term potentiation but not long-term depression. PLoS One 6: e17276.

Creighton, S.D., Mendell, A.L., Palmer, D., Kalisch, B.E., MacLusky, N.J., et al. (2019). Dissociable cognitive impairments in two strains of transgenic Alzheimer's disease mice revealed by a battery of object-based tests. Sci. Rep. 9, 57. 10.1038/s41598-018-37312-0.

Dieterich DC, Karpova A, Mikhaylova M, Zdobnova I, et al. (2008) Caldendrin-Jacob: a protein liaison that couples NMDA receptor signalling to the nucleus. PLoS Biol 6: e34

Gomes GM, Dalmolin GD, Bär J, et al. (2014) Inhibition of the polyamine system counteracts β-amyloid peptide-induced memory impairment in mice: involvement of extrasynaptic NMDA receptors. PLoS One 9: e99184

Grochowska KM, Kaushik R, Gomes GM, Raman R, Bär J, Bayraktar G, Samer S, Reyes-Resina I, Spilker C, Woo MS et al. (2020) A molecular mechanism by which amyloid-β induces inactivation of CREB in Alzheimer's Disease. bioRxiv: 2020.01.08.898304

Grochowska KM, Yuanxiang P, Bär J, et al. (2017) Posttranslational modification impact on the mechanism by which amyloid-β induces synaptic dysfunction. EMBO Rep 18: 962-981

Karpova A, Mikhaylova M, Bera S, Bär J, Reddy PP, Behnisch T, Rankovic V, Spilker C, Bethge P, Sahin J et al. (2013) Encoding and transducing the synaptic or extrasynaptic origin of NMDA receptor signals to the nucleus. Cell 152: 1119-33

Kindler S, Dieterich DC, et al. (2009) Dendritic mRNA targeting of Jacob and N-methyl-d-aspartate-induced nuclear translocation after calpain-mediated proteolysis. J Biol Chem 284: 25431-40 Melgarejo da Rosa M, Yuanxiang P, et al. (2016) Synaptic GluN2B/CaMKII-α Signaling Induces Synapto-Nuclear Transport of ERK and Jacob. Front Mol Neurosci 9: 66

Mikhaylova M, Karpova A, Bär J, Bethge P, YuanXiang P, Chen Y, Zuschratter W, Behnisch T, Kreutz MR (2014) Cellular distribution of the NMDA-receptor activated synapto-nuclear messenger Jacob in the rat brain. Brain Struct Funct 219: 843-60

Panayotis N, Karpova A, Kreutz MR, Fainzilber M (2015) Macromolecular transport in synapse to nucleus communication. Trends Neurosci 38: 108-16

Rönicke R, Mikhaylova M, Rönicke S, Meinhardt J, Schroder UH, Fändrich M, et al. (2011) Early neuronal dysfunction by amyloid β oligomers depends on activation of NR2B-containing NMDA receptors. Neurobiol Aging 32: 2219-28

Spilker C, Grochowska KM, Kreutz MR (2016a) What do we learn from the murine Jacob/Nsmf gene knockout for human disease? Rare Dis 4: e1241361

Spilker C, Nullmeier S, Grochowska KM, et al. (2016b) A Jacob/Nsmf Gene Knockout Results in Hippocampal Dysplasia and Impaired BDNF Signaling in Dendritogenesis. PLoS Genet 12: e1005907

## Claims

1. A compound nitarsone, or derivative or salt thereof for use in the treatment of a neurodegenerative disease in a subject.

2. The compound, or derivative or salt thereof for use according to claim 1, wherein the neurodegenerative disease is Alzheimer's disease (AD), dementia, Parkinson's disease (PD) or amyotrophic lateral sclerosis (ALS).

3. A pharmaceutical composition comprising the compound, or derivative or salt thereof according to any one of the preceding claims for use in the treatment of a neurodegenerative disease in a subject.

4. The pharmaceutical composition for use according claim 3, wherein the pharmaceutical composition is provided in a liquid or in a solid form.

5. The pharmaceutical composition for use according to claim 4, wherein the solid form is selected from the group comprising a tablet, an extended-release tablet, a coated tablet, a capsule, a dragee, a pill, a film, a lozenge and a powder.

6. The pharmaceutical composition for use according to claim 4, wherein the liquid form is selected from the group comprising a solution, an emulsion or a suspension.

7. The pharmaceutical composition for use according to any one of the claims 3-6, wherein the composition is administered orally, transmucosally, intravenously or intramuscular.

8. The pharmaceutical composition for use according to any one of the claims 3-7, wherein the composition is administered at least 1 time per day.

9. The pharmaceutical composition for use according to any one of the claims 3-8, wherein the composition is administered at least 1 time per day for multiple consecutive days.

10. The pharmaceutical composition for use according to any one of the claims 3-9, wherein the subject is showing one or more symptoms of a neurodegenerative disease, such as impaired memory, language, perceptual skills, attention, motor skills, orientation, problem solving and/or executive functional abilities.

11. The pharmaceutical composition for use according to any one of the claims 3-10, wherein the neurodegenerative disease is Alzheimer's disease (AD), dementia, Parkinson's disease (PD) or amyotrophic lateral sclerosis (ALS).

12. The pharmaceutical composition for use according to any one of the claims 3-11, wherein the neurodegenerative disease is Alzheimer's disease (AD).

13. The compound or derivative or salt thereof for use according to claim 1, wherein the compound is selected from the group comprising (4-nitrophenyl)arsonic acid, (4-nitrophenyl)stibonic acid, hydroxymethyl hydrogen(4-nitrophenyl)arsonate and hydroxymethyl hydrogen(4-nitrophenyl)stibonate.

14. The compound or derivative or salt thereof for use according to claim 1, wherein the compound is the nitarsone derivative 2-{[1-(4-nitrophenyl)ethyl]amino}ethan-1-ol (NPEAE) or a derivative or analogue thereof.

15. The compound for use according to claim 14, wherein the compound NPEAE, or derivative or analogue thereof is selected from the group comprising (S)-2-((1-(4-nitrophenyl)ethyl)amino)ethan-1 -ol, (S)-2-((hydroxyl(4-nitrophenyl)methyl)amino)ethan-1-ol, (R)-2-((amino(4-nitrophenyl)methyl)amino)ethan-1-ol, (R)-2-((2-hydroxyethyl)amino-2-(4-nitrophenyl)ethan-1-ol, (S)-2-((1-(4-nitrophenyl)amino)ethane-1-thiol, (S)-((2-mercaptoethyl)amino)(4-nitrophenyl)methanol, (R)-2-((amino(4-nitrophenyl)methyl)amino)ethane-1-thiol and (R)-2-((2-mercaptoethyl)amino)2-(4-nitrophenyl)ethan-1-ol.
